# EUROPEAN PATENT SPECIFICATION

(11) **EP 3 008 465 B1**
(45) Date of publication and mention of the grant of the patent: **07.08.2019**
(21) Application number: 14810249.4
(22) Date of filing: 13.06.2014
(51) Int. Cl.: G01N 33/487, G01N 33/68

(54) **METHOD OF SCREENING CANDIDATE BIOCHEMICAL ENTITIES TARGETING A TARGET BIOCHEMICAL ENTITY**
VERFAHREN ZUM SCREENING VON BIOCHEMISCHEN KANDIDATENENTITÄTEN ZUR ANZIELUNG EINER BIOCHEMISCHEN ZIELENTITÄT
PROCÉDÉ DE CRIBLAGE D'ENTITÉS BIOCHIMIQUES CANDIDATES CIBLANT UNE ENTITÉ BIOCHIMIQUE CIBLE

(30) Priority: 13.06.2013 US 201361834782 P
(43) Date of publication of application: 20.04.2016
(73) Proprietor: Biodesy, Inc., South San Francisco, California 94080 (US)
(72) Inventor: SALAFSKY, Joshua, San Francisco, CA 94110 (US); MOREE III, Carl, Benton, Redwood City, CA 94061 (US)
(74) Representative: Avidity IP
(86) International application number: PCT/US2014/042428
(87) International publication number: WO 2014/201435

(56) References cited:
- WO-A1-2012/129347
- WO-A1-2013/162654
- US-A1- 2003 224 390
- US-A1- 2005 107 472
- US-A1- 2010 068 144
- US-A1- 2010 120 164
- US-A1- 2013 108 549
- Salafsky Joshua: "Detection Method for Conformational Change", Genetic Engineering & Biotechnology News, 15 April 2009 (2009-04-15), XP055303299, Retrieved from the Internet: URL:http://www.genengnews.com/keywordsandt ools/print/1/12798/ [retrieved on 2016-09-16]
- JIHUA WANG ET AL: "Novel Strategies for Drug Discovery Based on Intrinsically Disordered Proteins (IDPs)", INTERNATIONAL JOURNAL OF MOLECULAR SCIENCES, vol. 12, no. 12, 17 May 2011 (2011-05-17), pages 3205-3219, XP055303315, DOI: 10.3390/ijms12053205
- FRANCESCO VANZI ET AL: "Protein conformation and molecular order probed by second-harmonic-generation microscopy", JOURNAL OF BIOMEDICAL OPTICS, vol. 17, no. 6, 18 June 2012 (2012-06-18), page 060901, XP055303319, US ISSN: 1083-3668, DOI: 10.1117/1.JBO.17.6.060901
- BEN MOREE ET AL: "Small Molecules Detected by Second-Harmonic Generation Modulate the Conformation of Monomeric [alpha]-Synuclein and Reduce its Aggregation in Cells", JOURNAL OF BIOLOGICAL CHEMISTRY, 22 September 2015 (2015-09-22), XP055303307, US ISSN: 0021-9258, DOI: 10.1074/jbc.M114.636027

## Description

### BACKGROUND OF THE INVENTION

The effort to discover drugs to treat diseases has been revolutionized by high-throughput technologies in genomics and biological screening, combinatorial chemistry, and computer technology. However, scientists must still sift through enormous numbers of potential drug candidates in search for an effective drug.

Using conventional screening techniques to identify allosteric modulators is a difficult task. Only about 15% of all proteins have a sufficiently deep pocket at the active site to support binding of an inhibitor more tightly than the native ligand. Activity assays for targets such as enzymes can and have been used to identify allosteric modulators, but the mechanism of action can be laborious to determine, since the assays typically involve multiple proteins and the modulator's action could be directed to ones other than the target of interest. Screening of such modulators involves sorting competitive inhibitors from non-competitive inhibitors and then allosteric modulators from non-specific modulators (aggregators, high-stoichiometric binders, etc.) in the latter group
Salafsky (Genetic Engineering & Biotechnology News; Apr 15, 2009, vol 29, no 8) discloses second harmonic generation as a means for detecting conformation changes in proteins.

### BRIEF SUMMARY

According to the present invention there is provided a method of screening biochemical entities, the method comprising:
(a) providing a library comprising 6 or more candidate biochemical entities;
(b) measuring a first conformational state of an intrinsically disordered protein, thereby generating a baseline;
(c) contacting each of the candidate biochemical entities with the same intrinsically disordered protein;
(d) measuring a second conformational state of the intrinsically disordered protein;
(e) determining a signature for at least a first and a second candidate biochemical entity using the second conformational state
(f) comparing the signature of the first candidate biochemical entity to the signature of the second candidate biochemical entity; and
(g) selecting, based on the comparison in (f), one or more biochemical entities from the candidate biochemical entities,
where the candidate biochemical entities are small molecules that share a common scaffold core, and where the measuring steps comprise measurements of second harmonic generation (SHG).

Preferred embodiments of the invention are provided in the dependent claims in the appended claims.

The disclosure provides methods of screening drug candidates targeting a target biomolecule, comprising selecting a drug candidate based on a signal difference between a first signal and a second signal. The signal difference indicates a difference in an *in vivo* or *in vitro* pharmacological property. The first signal is produced upon binding between the target biomolecule and a first candidate by contacting the target biomolecule with the first candidate, and the second signal is produced upon binding between the target biomolecule and a second candidate by contacting the target biomolecule with the second candidate. The first signal and the second signal are generated by the second harmonic generation-active moiety using a surface selective technique. The first molecule and the second molecule have a similar scaffold core targeting a binding site in the target biomolecule. Optionally, prior to the selecting step, the method further comprises contacting the target biomolecule with the first candidate, detecting the first signal upon binding between the target biomolecule and the first candidate; and contacting the target biomolecule with the second candidate, detecting the second signal upon binding between the target biomolecule and the second candidate. Optionally, the first molecule and the second molecule have a same scaffold core targeting a binding site in the target biomolecule. Optionally, the method further comprises generating a signal difference between the first signal and the second signal. Optionally, the signal difference is 1% or greater above noise level. Optionally, the signal difference is 10% or greater above noise level. Optionally, the second candidate is a reference molecule associated with a desired *in vivo* or *in vitro* pharmacological property. Optionally, a positive signal difference indicates an improvement on the *in vivo* or *in vitro* pharmacological property. Optionally, a negative signal difference indicates an improvement on the *in vivo* or *in vitro* pharmacological property. Optionally, the signal is concentration-normalized. Optionally, the signal can be normalized second harmonic generation intensity. Optionally, the methods further comprise screening the selected drug candidate in an animal.

The disclosure also provides methods of screening drug candidates targeting a target biomolecule, comprising selecting a drug candidate based on a signal produced upon contacting a binding complex of the target biomolecule and a candidate molecule with a reference molecule targeting the same binding site as the candidate molecule. The reference molecule is associated with an *in vivo* or *in vitro* pharmacological property. The signal is generated by the second harmonic generation-active moiety using a surface selective technique. The candidate molecule and the reference molecule have a same or similar scaffold core targeting a binding site in the target biomolecule. Optionally, prior to the selecting step, the method further comprises, contacting the target biomolecule with the candidate molecule, wherein the candidate molecule binds to a binding site in the target biomolecule thereby forming the binding complex; contacting the binding complex with the reference molecule; and detecting a signal upon contacting the binding complex with the reference molecule. Optionally, a signal of no more than 1% above noise level indicates a comparable or increased *in vivo* or *in vitro* pharmacological property as compared to the reference molecule. Optionally, a signal of no more than 10% above noise level indicates a comparable or increased *in vivo* or *in vitro* pharmacological property as compared to the reference molecule. Optionally, the signal is concentration-normalized. Optionally, the signal can be normalized second harmonic generation intensity. Optionally, the methods further comprise screening the selected drug candidate in an animal.

The disclosure also provides methods of screening drug candidates targeting a target biomolecule, comprising selecting a drug candidate based on a signal produced upon contacting a binding complex of the target biomolecule and a reference molecule with a candidate molecule targeting the same binding site as the reference molecule. The reference molecule is associated with an *in vivo* or *in vitro* pharmacological property. The signal is generated by the second harmonic generation-active moiety using a surface selective technique. The first candidate and the reference molecule have a same or similar scaffold core targeting a binding site in the target biomolecule. Optionally, prior to the selecting step, the method further comprises contacting the target biomolecule with the reference molecule, wherein the reference molecule binds to a binding site in the target biomolecule thereby forming the binding complex; contacting the binding complex with the candidate molecule; and detecting a signal upon contacting the binding complex with the candidate molecule. Optionally, a signal of more than 1% above noise level indicates a comparable or increased *in vivo* or *in vitro* pharmacological property as compared to the reference molecule. Optionally, a signal of more than 10% above noise level indicates a comparable or increased *in vivo* or *in vitro* pharmacological property as compared to the reference molecule. Optionally, the signal is concentration-normalized. Optionally, the signal can be normalized second harmonic generation intensity. Optionally, the methods further comprise screening the selected drug candidate in an animal.

The disclosure also provides methods for identifying a binding moiety for a target bio molecule, comprising identifying the binding moiety by comparing the structures of the first candidate and the second candidate, which are selected based on a signal difference between a first signal and a second signal. The signal difference indicates the presence of the binding moiety in the first molecule, and the absence of the binding moiety in the second molecule. The first signal is produced upon binding between the target biomolecule and a first molecule by contacting the target biomolecule with the first molecule. The second signal is produced upon binding between the target biomolecule and a second molecule by contacting the target biomolecule with the second molecule. The first signal and the second signal are generated by the second harmonic generation-active moiety using a surface selective technique. The first molecule and the second molecule have a same or similar scaffold core targeting a binding site in the target biomolecule. Optionally, the signal difference indicates a difference in an *in vivo* or *in vitro* pharmacological property. Optionally, the signal difference is 1% or greater above noise level. Optionally, the signal difference is 10% or greater above noise level. Optionally, prior to the identifying step, the method further comprises contacting the target biomolecule with the first molecule, detecting the first signal upon binding between the target biomolecule and the first molecule; and contacting the target biomolecule with the second molecule, detecting the second signal upon binding between the target biomolecule and the second molecule. Optionally, the method further comprises generating the signal difference between the first signal and the second signal. Optionally, the signal is concentration-normalized. Optionally, the signal can be normalized second harmonic generation intensity.

The disclosure also provides methods for identifying a binding moiety for a target biomolecule, comprising identifying the binding moiety by comparing the structures of the candidate molecule and the reference molecule, which are selected based on a signal produced upon contacting a binding complex of the target biomolecule and the candidate molecule with a reference molecule targeting the same binding site as the candidate molecule. The signal indicates the presence of the binding moiety in the reference molecule, and the absence of the binding moiety in the candidate molecule. The signal is generated by the second harmonic generation-active moiety using a surface selective technique. The candidate molecule and the reference molecule have a same or similar scaffold core targeting a binding site in the target biomolecule. Optionally, a signal of no more than 1% above noise level indicates a comparable or increased *in vivo* or *in vitro* pharmacological property as compared to the reference molecule. Optionally, a signal of no more than 10% above noise level indicates a comparable or increased *in vivo* or *in vitro* pharmacological property as compared to the reference molecule. Optionally, prior to the identifying step, the method further comprises contacting the target biomolecule with the candidate molecule, wherein the candidate molecule binds to a binding site in the target biomolecule thereby forming the binding complex; contacting the binding complex with the reference molecule; and detecting the signal upon contacting the binding complex with a reference molecule. Optionally, the signal is concentration-normalized. Optionally, the signal can be normalized second harmonic generation intensity.

The disclosure further provides methods for identifying a binding moiety for a target biomolecule, comprising identifying the binding moiety by comparing the structures of the candidate molecule and the reference molecule, which are selected based on a signal produced upon contacting a binding complex of the target biomolecule and the reference molecule with a candidate molecule targeting the same binding site as the reference molecule. The signal indicates the presence of the binding moiety in the reference molecule, and the absence of the binding moiety in the candidate molecule. The signal is generated by the second harmonic generation-active moiety using a surface selective technique. The candidate molecule and the reference molecule have a same or similar scaffold core targeting a binding site in the target biomolecule. Optionally, a signal of more than 1% above noise level indicates a comparable or increased *in vivo* or *in vitro* pharmacological property as compared to the reference molecule. Optionally, a signal of more than 10% above noise level indicates a comparable or increased *in vivo* or *in vitro* pharmacological property as compared to the reference molecule. Optionally, prior to the identifying step, the method further comprising contacting the target biomolecule with the reference molecule, wherein the reference molecule binds to a binding site in the target biomolecule thereby forming the binding complex; contacting the binding complex with the candidate molecule; and detecting the signal upon contacting the binding complex with the candidate molecule. Optionally, the signal is concentration-normalized. Optionally, the signal can be normalized second harmonic generation intensity.

Optionally, the *in vivo* pharmacological property is therapeutic efficacy. Optionally, the *in vivo* pharmacological property is a clinical efficacy property. Optionally, the *in vivo* pharmacological property is a pharmacokinetic property. Optionally, the *in vivo* pharmacological property is a clinical toxicity property. Optionally, the *in vivo* pharmacological property is a preclinical toxicity property. Optionally, the *in vivo* pharmacological property is a pharmacodynamics property. Optionally, the *in vivo* pharmacological variable is therapeutic index. Optionally, the binding site is an active site. Optionally, the binding site is an allosteric binding site. Optionally, the target biomolecule is a protein. Optionally, the target biomolecule is a DNA. Optionally, the target biomolecule is alpha synuclein. Optionally, the methods further comprise screening the selected drug candidate in an animal. Optionally, the drug candidate is a small molecule. Optionally, the drug candidate is a protein. Optionally, the drug candidate is an antibody. Optionally, the drug candidate is a biobetter or biosimilar drug. Optionally, the drug candidates are phthalocyanine tetrasulfonate derivatives or analogs. Optionally, the drug candidates are antibodies to alpha synuclein. Optionally, the drug candidates are bioequivalents of solanezumab or bapineuzumab.

The disclosure provides a method of screening biochemical entities, the method comprising: (a) providing a library comprising 6 or more candidate biochemical entities, wherein the 6 or more candidate biochemical entities are structurally similar as determined using one or more structural similarity parameters selected from the group consisting of: (i) molecular weight within 150 g/mol of each of the candidate biochemical entities; (ii) molecular weight within 30% of each of the candidate biochemical entities; (iii) chemical formula to within 15 atoms of each of the candidate biochemical entities; (iv) a difference in total number of atoms of less than 15 between each of the candidate biochemical entities; (v) a difference in number of carbon atoms of less than 5; (vi) a difference in number of hydrogen atoms of less than 5; (vii) a difference in number of nitrogen atoms of less than 5; (viii) a difference in number of oxygen atoms of less than 5; (ix) a difference in number of sulfur atoms of less than 5; (x) a polarity as measured by log D within 10% of each of the candidate biochemical entities; (xi) a polarity as measured by log P (partition coefficient) within 10% of each of the candidate biochemical entities; (xii) a polarity as measured by PSA (polar surface area) within 10% of each of the candidate biochemical entities; (xiii) an identical scaffold core; (xiv) a chemical similarity as measured by graph theory; (xv) a Tanimoto (or Jaccard) coefficient T greater than 0.85; (xvi) a difference in number of aromatic groups of less than 3; (xvii) a difference in number of heterocyclic groups of less than 3; (xviii) a difference in number of monocyclic groups of less than 3; (xix) a difference in number of fused ring systems of less than 3; and (xx) a difference in number of double bonds of less than 3; (b) measuring a first conformational state of a target biochemical entity, thereby generating a baseline; (c) contacting each of the candidate biochemical entities with a same target biochemical entity; (d) measuring a second conformational state of the target biochemical entity; (e) determining a signature of the target biochemical entity using the second conformational state; and (f) selecting one or more biochemical entities from the candidate biochemical entities based on the signature.

In some cases, measuring the first or second conformational state can use total internal reflection (TIR). In some cases, measuring the first or second conformational state can use second harmonic generation (SHG).

In some cases, determining the signature can comprise measuring a kinetic (real time) change in conformational state of the target biochemical entity. In other cases, determining the signature can comprise measuring an end-point change in conformational state of the target biochemical entity. I yet other cases, determining the signature can comprise measuring a signal intensity of the signal. In further cases, the determining of the signature uses the baseline.

In some cases, the method can further comprise analyzing, using a computer-executable program, the 6 or more candidate biochemical entities based on one or more structural similarity parameters. In other cases, the method can further comprise analyzing, using a computer-executable program, the signature.

Each of the candidate biochemical entities can produce a different change in conformational state of the target biochemical entity. In addition, upon the contacting in step (c), each of the candidate biochemical entities can generate a distinct signature.

The method can further comprise comparing the change in conformational state of the target biochemical entity to a change in conformational state produced by a known conformational modulator. The known conformational modulator can be a known drug. The method can further comprise selecting one or more biochemical entities based on the change in conformational state that is most similar to the change in conformational state induced by the known conformational modulator. In some cases, the change in conformational state produced by the known conformational modulator can correlate to a pharmacological property or a functional effect.

In some cases, at least two of the candidate biochemical entities have a similar pharmacological property or functional effect. In other cases, at least two of the candidate biochemical entities do not have a similar pharmacological property or functional effect. Further, one or more biochemical entities can be selected based on a correlation of the signature to a known pharmacological property or a known functional effect.

The first conformational state can be measured after the target biochemical entity is incubated with an agent. For example, the agent can be a candidate biochemical entity from the library.

The method can further comprise measuring a signature parameter selected from the group consisting of: i) a change in magnitude of the signature relative to the baseline; ii) a change in direction of the signature relative to the baseline; and iii) a change in rate of a signal per unit time of the signature upon the contacting of the candidate biochemical entity. In addition, the signature generated by a first candidate biochemical entity and the signature generated by a second candidate biochemical entity can be similar, as determined using one or more signature similarity parameters selected from the group consisting of: i) the change in magnitude of the signature relative to the baseline upon contacting the first candidate biochemical entity is within 50% of the change in magnitude of the signature relative to the baseline upon contacting the second candidate biochemical entity; ii) the change in direction of the signature relative to the baseline upon contacting the first candidate biochemical entity is the same as the change in direction of the signature relative to the baseline upon contacting the second candidate biochemical entity; and iii) the change in rate of a signal per unit time of the signature upon contacting the first candidate biochemical entity is within two-fold of the change in rate of a signal per unit time of the signature upon contacting the second candidate biochemical entity.

The disclosure also provides a method of screening biochemical entities, the method comprising: (a) providing a library comprising 6 or more candidate biochemical entities; (b) measuring a first conformational state of the target biochemical entity, thereby generating a baseline; (c) contacting each of the candidate biochemical entities with a same target biochemical entity; (d) measuring a second conformational state of the target biochemical entity; (e) determining a signature for at least a first and a second candidate biochemical entity using the second conformational state; (f) comparing the signature of the first biochemical entity to the signature of the second biochemical entity; and (g) selecting, based on the comparison in (f), one or more biochemical entities from the candidate biochemical entities.

In some cases, the 6 or more candidate biochemical entities are structurally similar as determined using one or more structural similarity parameters selected from the group consisting of: (i) molecular weight within 150 g/mol of each of the candidate biochemical entities; (ii) molecular weight within 30% of each of the candidate biochemical entities ? not clear; (iii) chemical formula to within 15 atoms of each of the candidate biochemical entities; (iv) a difference in total number of atoms of less than 15 between each of the candidate biochemical entities; (v) a difference in number of carbon atoms of less than 5;(vi) a difference in number of hydrogen atoms of less than 5; (vii) a difference in number of nitrogen atoms of less than 5; (viii) a difference in number of oxygen atoms of less than 5; (ix) a difference in number of sulfur atoms of less than 5; (x) a polarity as measured by log D within 10% of each of the candidate biochemical entities; (xi) a polarity as measured by log P (partition coefficient) within 10% of each of the candidate biochemical entities; (xii) a polarity as measured by PSA (polar surface area) within 10% of each of the candidate biochemical entities; (xiii) an identical scaffold core; (xiv) a chemical similarity as measured by graph theory; (xv) a Tanimoto (or Jaccard) coefficient T greater than 0.85; (xvi) a difference in number of aromatic groups of less than 3; (xvii) a difference in number of heterocyclic groups of less than 3; (xviii) a difference in number of monocyclic groups of less than 3; (xix) a difference in number of fused ring systems of less than 3; and (xx) a difference in number of double bonds of less than 3;

The method can further comprise analyzing, using a computer-executable program, the signature. Each of the candidate biochemical entities can produce a different change in conformational state of the target biochemical entity. In some cases, upon the contacting in (c), each of the candidate biochemical entities can generate a distinct signature. In some cases the signature of one or more of the biochemical entities correlates to a known pharmacological property or a known functional effect. In further cases, at least two of the candidate biochemical entities have a similar pharmacological property or functional effect.

The method can further comprising measuring a parameter selected from the group consisting of: i) a change in magnitude of the signature relative to the baseline; ii) a change in direction of the signature relative to the baseline; and iii) a change in rate of a signal per unit time of the signature upon the contacting of the candidate biochemical entity. In addition, the signature generated by a first candidate biochemical entity and the signature generated by a second candidate biochemical entity can be similar, as determined using one or more signature similarity parameters selected from the group consisting of: i) the change in magnitude of the signature relative to the baseline upon contacting the first candidate biochemical entity is within 50% of the change in magnitude of the signature relative to the baseline upon contacting the second candidate biochemical entity; ii) the change in direction of the signature relative to the baseline upon contacting the first candidate biochemical entity is the same as the change in direction of the signature relative to the baseline upon contacting the second candidate biochemical entity; and iii) the change in rate of a signal per unit time of the signature upon contacting the first candidate biochemical entity is within two-fold of the change in rate of a signal per unit time of the signature upon contacting the second candidate biochemical entity.

The present disclosure further provides a method of cataloging biochemical entities, the method comprising: (a) providing a library of biochemical entities, wherein each of the biochemical entities comprises a distinct molecular structure; (b) contacting at least one biochemical entity in the library with a same target biochemical entity; (c) measuring a conformational state of the target biochemical entity; (d) determining a signature using the conformational state; and (e) generating a structure-activity relationship (SAR) catalog using the signature.

In some cases, generating the SAR can comprise correlating the signature with a binding property, a functional property, an efficacy, a potency, a selectivity, or a combination thereof. In some cases, generating the SAR can comprise correlating the signature to one or more structural parameters of the candidate biochemical entities, wherein said structural parameters can be selected from the group consisting of: molecular weight, chemical formula, number of total atoms, number of carbon atoms, number of hydrogen atoms, number of nitrogen atoms, number of oxygen atoms, number of sulfur atoms, polarity as measured by log D, polarity as measured by log P, polarity as measured by PSA (polar surface area), the scaffold core, chemical similarity as measured by graph theory, Tanimoto (or Jaccard) coefficient T, number of aromatic groups, number of heterocyclic groups, number of monocyclic groups, number of fused ring systems, and number of double bonds.

The method can further comprise measuring the conformational state of the target biochemical prior to the contacting, thereby generating a baseline. The method can further comprise analyzing the SAR to determine a binding moiety within at least one of the biochemical entities that is associated with a pharmacological property or a functional effect.

In some cases, determining the signature can comprise measuring a kinetic (real time) change in the conformational state of the target biochemical entity. In other cases, determining the signature comprises measuring an end-point change in the conformational state of the target biochemical entity. The signature can indicate a change in the conformational state of the target biochemical entity. The method can further comprise analyzing, using a computer-executable program, the signature.

The library of biochemical entities can comprise drug candidates. The target biochemical entity can be a drug target. The drug target can have a known pharmacological property or a known functional effect. The method can further comprise selecting one or more biochemical entities based on a correlation of the SAR catalog or the signature to a known pharmacological property or a known functional effect.

The method can further comprise measuring a signature parameter selected from the group consisting of: i) a change in magnitude of the signature relative to the baseline; ii) a change in direction of the signature relative to the baseline; and iii) a change in rate of a signal per unit time of the signature upon the contacting of the candidate biochemical entity. In addition, the signature generated by a first candidate biochemical entity and the signature generated by a second candidate biochemical entity can be similar, as determined using one or more signature similarity parameters selected from the group consisting of: i) the change in magnitude of the signature relative to the baseline upon contacting the first candidate biochemical entity is within 50% of the change in magnitude of the signature relative to the baseline upon contacting the second candidate biochemical entity; ii) the change in direction of the signature relative to the baseline upon contacting the first candidate biochemical entity is the same as the change in direction of the signature relative to the baseline upon contacting the second candidate biochemical entity; and iii) the change in rate of a signal per unit time of the signature upon contacting the first candidate biochemical entity is within two-fold of the change in rate of a signal per unit time of the signature upon contacting the second candidate biochemical entity.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

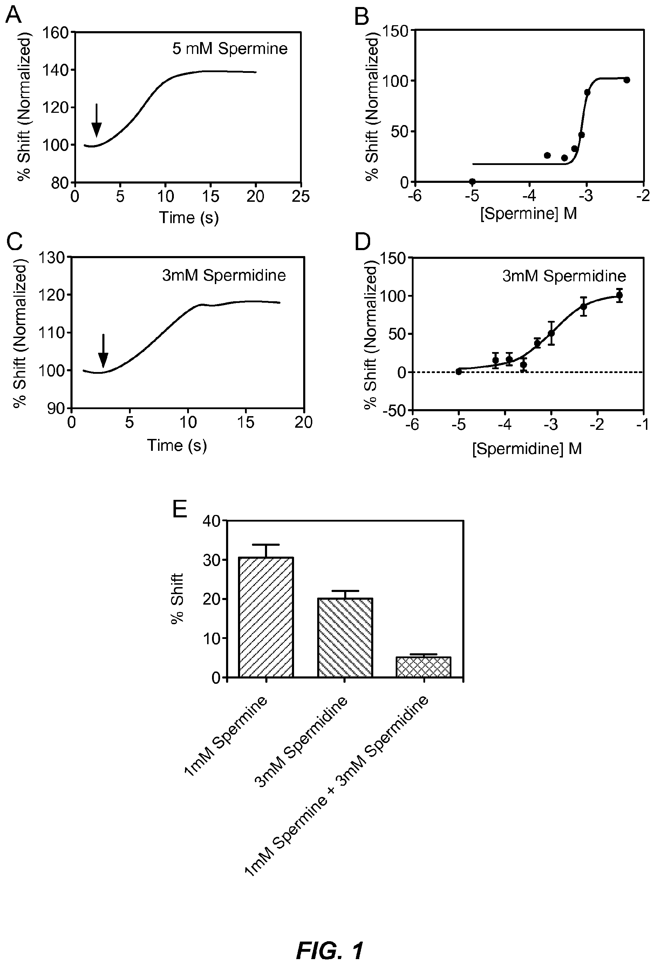

**Figure 1A** shows that spermine and spermidine induce a conformational change in alpha-synuclein as measured by second harmonic generation. A representative trace showing the SHG response in real time upon exposure of alpha-synuclein to 5mM spermine. The arrow denotes spermine addition. The change in SHG intensity is normalized to the value just prior to injection.
**Figure 1B** shows the dose response curve of spermine on alpha-synuclein conformation as measured by SHG. The dose curve (plotted on the log scale) was determined by adding spermine at the given concentrations to 5µM alpha-synuclein and measuring the change in SHG intensity. The change in SHG intensity for each concentration was quantified as percent shift (also "change" herein).
**Figure 1C** provides a representative trace showing the SHG response in real time upon exposure of alpha-synuclein to 3mM spermidine. The arrow denotes spermidine addition. The change in SHG intensity is normalized to the value just prior to injection.
**Figure 1D** shows the dose response curve of spermidine on alpha-synuclein conformation as measured by SHG. The dose curve (plotted on the log scale) was determined by adding sperimidine at the given concentrations to 5µM alpha-synuclein and measuring the change in SHG intensity. The change in SHG intensity for each concentration was quantified as percent shift. Error bars = SEM. N = 3.
**Figure 1E** is a quantification of the Spermine-Spermidine Competition Assay. The 1mM Spermine and 3mM Spermidine controls are shown in the left and middle columns, respectively. Addition of 3mM Spermidine to samples preincubated with 1mM spermine is shown in the right column. Change in SHG intensity plotted as percent shift. Error bars = SEM. N = 4.

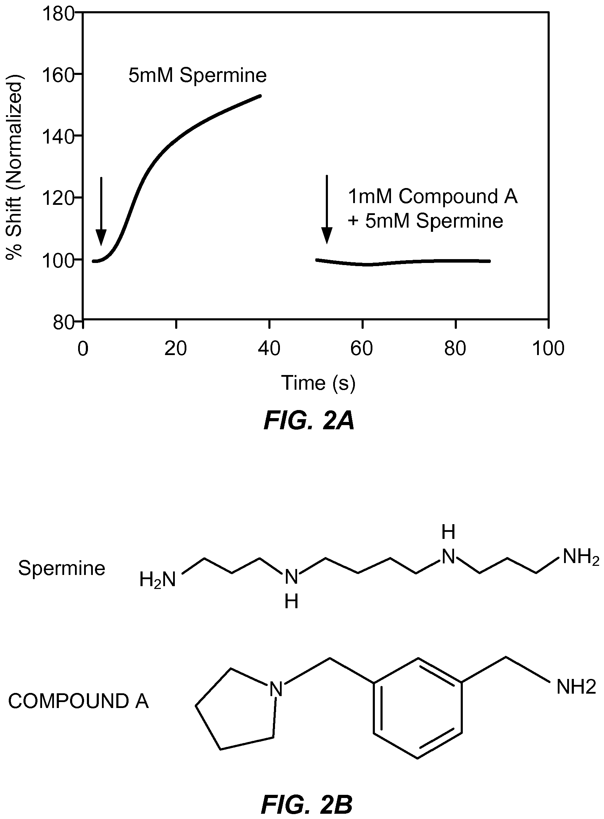

**Figure 2A** shows a second harmonic generation based screen of the Maybridge Ro3 fragment library. Representative traces showing the SHG response used to screen the Maybridge Ro3 fragment library for inhibitors of alpha-synuclein's spermine induced conformational change. On the left, a typical response for alpha-synuclein exposure to 5mM spermine. On the right, the response of alpha-synuclein preincubated with 1mM COMPOUND A upon exposure to 5mM spermine, indicating a negligible change over baseline.
**Figure 2B** schematically illustrates the chemical structures of spermine (top) and the compound COMPOUND A (bottom).

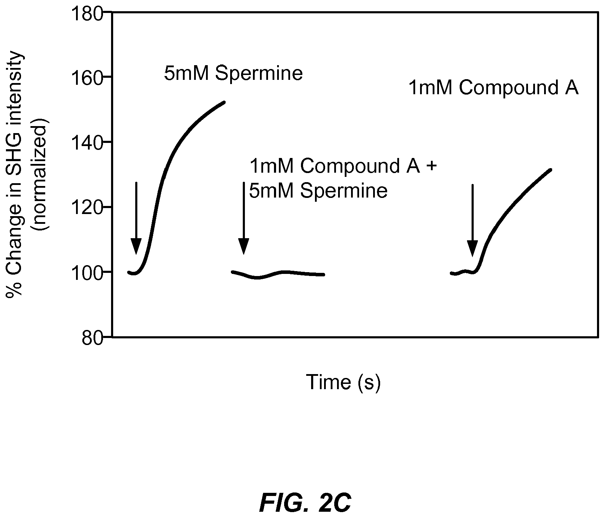

**Figure 2C** provides representative traces showing the SHG response used to screen the Maybridge Ro3 fragment library for inhibitors of alpha-synuclein's spermine induced conformational change. On the left, a representative trace showing the typical SHG response for alpha-synuclein upon exposure to 5mM spermine. In the middle, the SHG response of alpha-synuclein preincubated with 1mM COMPOUND A after exposure to 5mM spermine, indicating a negligible change over baseline. On the right, a representative trace showing the SHG response in real time upon exposure of alpha-synuclein to 1mM COMPOUND A. The arrows denote COMPOUND A addition. The change in SHG intensity is normalized to the value just prior to injection.

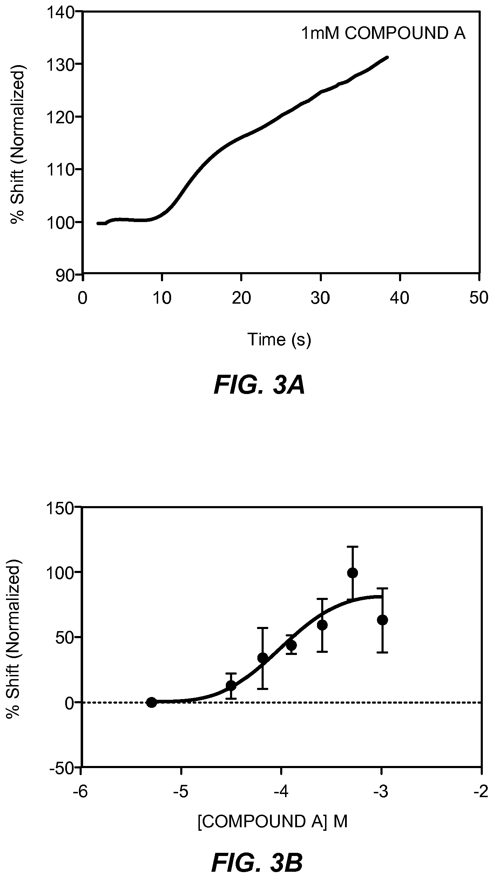

**Figure 3A** illustrates the characterization of COMPOUND A. A representative trace showing the SHG response in real time upon exposure of alpha-synuclein to 1mM COMPOUND A. The arrow denotes COMPOUND A addition. The change in SHG intensity is normalized to the value just prior to injection.
**Figure 3B** provides the dose response curve of COMPOUND A on alpha-synuclein conformation as measured by SHG. The dose curve (plotted on the log scale) was determined by adding COMPOUND A at the given concentrations to 5µM alpha-synuclein and measuring the change in SHG intensity. The change in SHG intensity for each concentration was quantified as percent shift. Error bars = SEM. N = 3.

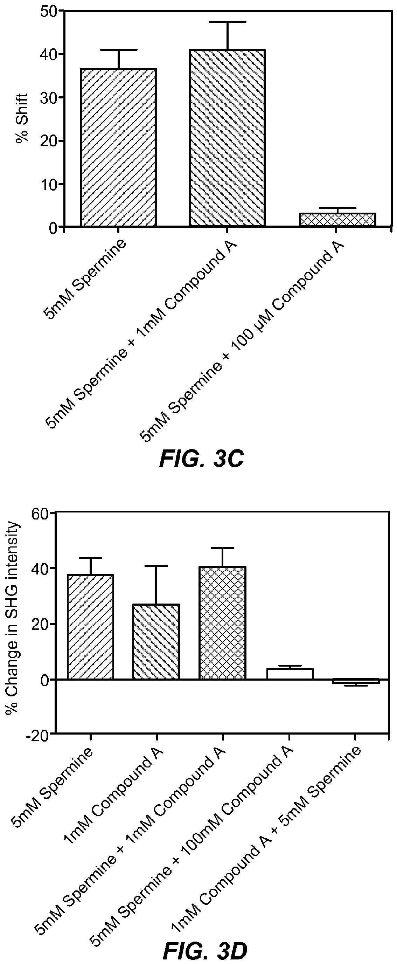

**Figure 3C** is a quantification of the Spermine- COMPOUND A Competition Assay. The 5mM Spermine control is shown on the left. Addition of either 1mM or 100µM COMPOUND A to samples preincubated with spermine are shown in the middle and right columns, respectively. Change in SHG intensity plotted as percent shift (also "change" herein).
**Figure 3D** is a quantification of the spermine-COMPOUND A Competition assay. The typical percent change in SHG intensity for both 5mM spermine and 1mM COMPOUND A only are shown in the first and second columns, respectively. The change in SHG intensity upon addition of either 1mM or 100µM COMPOUND A to samples preincubated with spermine are shown in the third and fourth columns, respectively. The change in SHG intensity upon addition of 5mM Spermine after incubation with 1mM COMPOUND A is shown in the fifth column. Change in SHG intensity plotted as percent change. Error bars = SEM. N = 3.

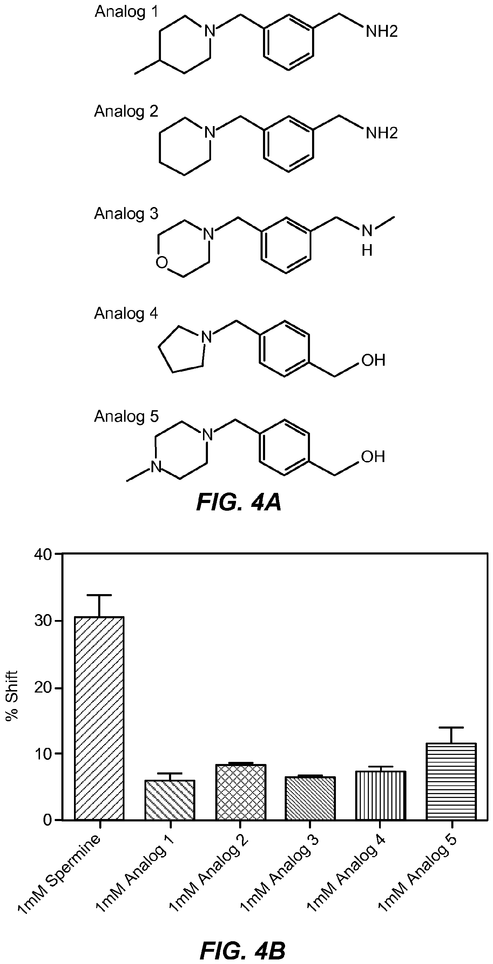

**Figure 4A** shows that COMPOUND A is a highly specific inhibitor of the alpha-synuclien's spermine induced conformational change. Chemical Structures of the top five scoring COMPOUND A analogs from the Maybridge Ro3 library as predicted by the program SimFinder.
**Figure 4B** is a quantification of the conformational change of alpha-synuclein upon exposure to the top five scoring chemical analogs of COMPOUND A. Each analog was added to a well containing 5µM alpha synuclein and the change in SHG intensity was measured. A representative data set indicating the typical SHG response for 1mM Spermine addition is included for comparison purposes. Changes in SHG intensity plotted as percent shift. Error bars = SEM. N = 3.

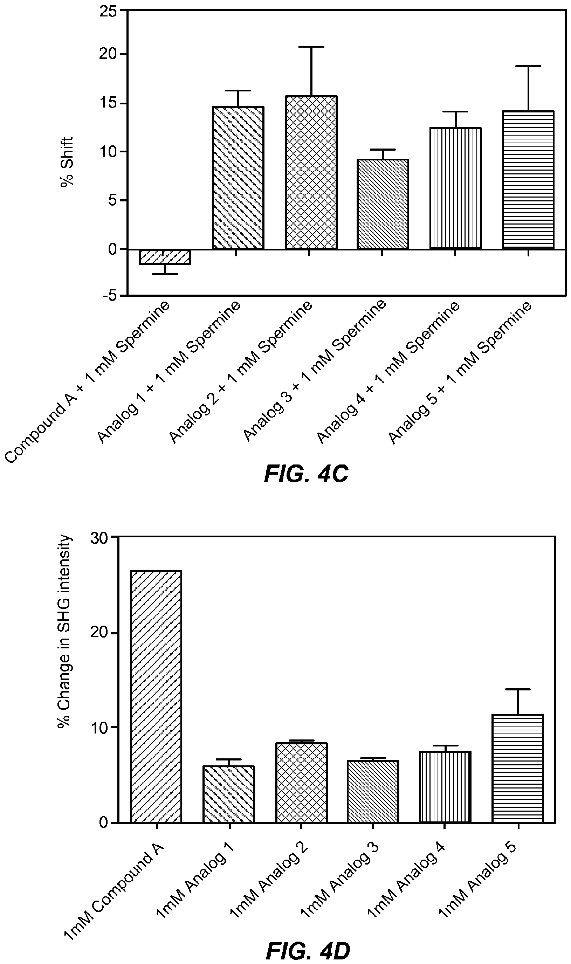

**Figure 4C** is a quantification of the conformational change of alpha-synuclein in the Analog-Spermine Competition Assay. The indicated COMPOUND A analog was preincubated with 5µM alpha synuclein for 25 minutes and the change in SHG intensity upon exposure to 1mM spermine was measured. A representative data set indicating the typical SHG response from the COMPOUND A + 1mM Spermine competition assay is included for comparison purposes. Changes in SHG intensity plotted as percent shift.
**Figure 4D** is a quantification of the conformational change of alpha-synuclein upon exposure to the top five scoring chemical analogs of COMPOUND A. Each analog was added at 1mM final concentration to a well containing 5µM alpha-synuclein and the change in SHG intensity was measured. A representative data set indicating the typical SHG response for 1mM COMPOUND A addition is included for comparison purposes. Changes in SHG intensity plotted as percent change. Error bars = SEM. N = 3.

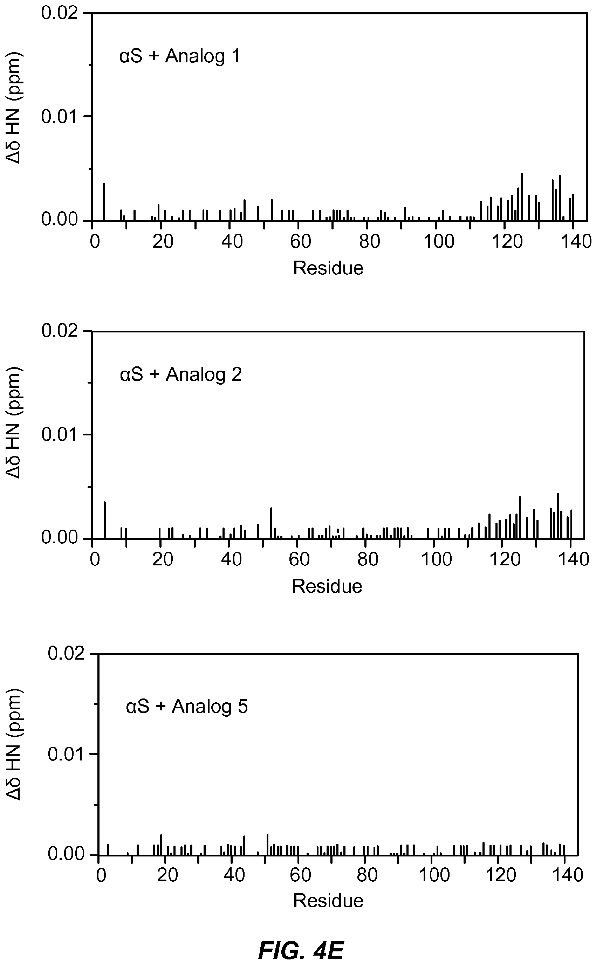

**Figure 4E** provides chemical shift perturbation analysis of ¹H-¹⁵N resonances of alpha-synuclein in the presence of analog Analog 1 (left), Analog 2 (middle), and Analog 5 (right) at a molar ratio of 1:12. Error bars = SEM. N = 3.

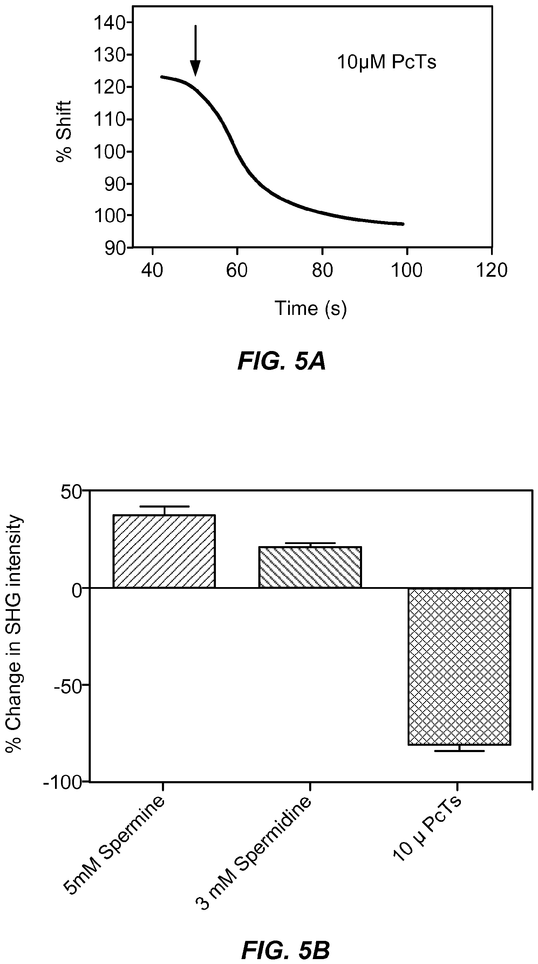

**Figure 5A** shows that phthalocyanine tetrasulfonate, an inhibitor of alpha-synuclein aggregation, produces a distinct SHG signature. A representative trace showing the SHG response in real time upon exposure of alpha-synuclein to 10µM PcTs. Arrow indicates time of PcTs addition. Changes in SHG intensity plotted as percent shift. Error = SEM. N = 5.
**Figure 5B** is a quantification of change in SHG intensity of alpha-synuclein upon exposure to Spermine, Spermidine, and Phthalocyanine Tetrasulfonate. 5mM spermine is shown on the left, 3mM Spermidine is shown in the middle, and 10µM PcTS is shown on the right. The change in SHG intensity plotted as percent change. Error bars = SEM. N = 4.

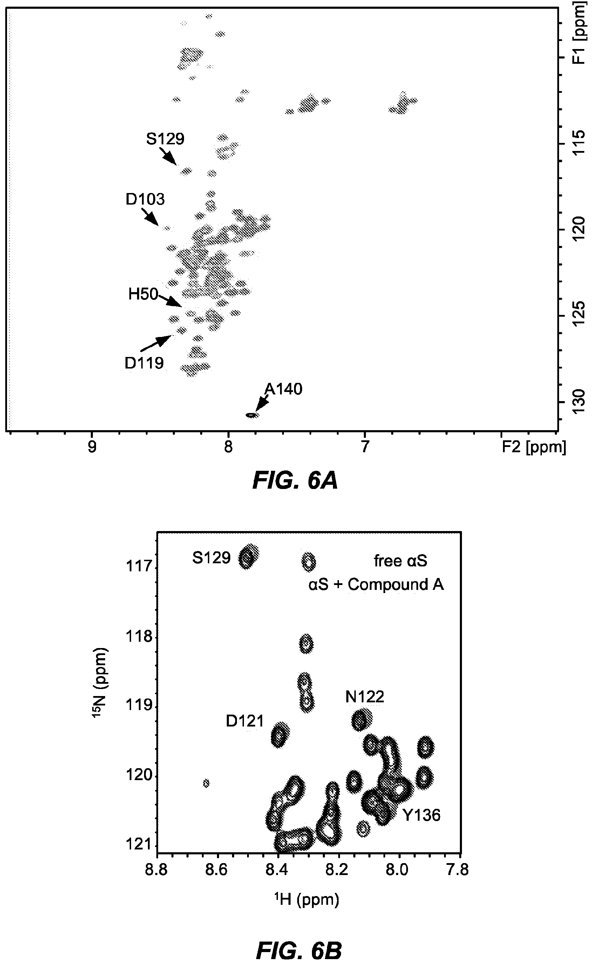

**Figure 6A** shows that 2-D HSQC NMR indicates that COMPOUND A binds to the same site as spermine on the C-terminal region of the protein.
**Figure 6B** is a detail of the superposition of 1H-15N NMR spectra of 100 µM alpha-synuclein in the absence (black) and presence (red) of COMPOUND A.

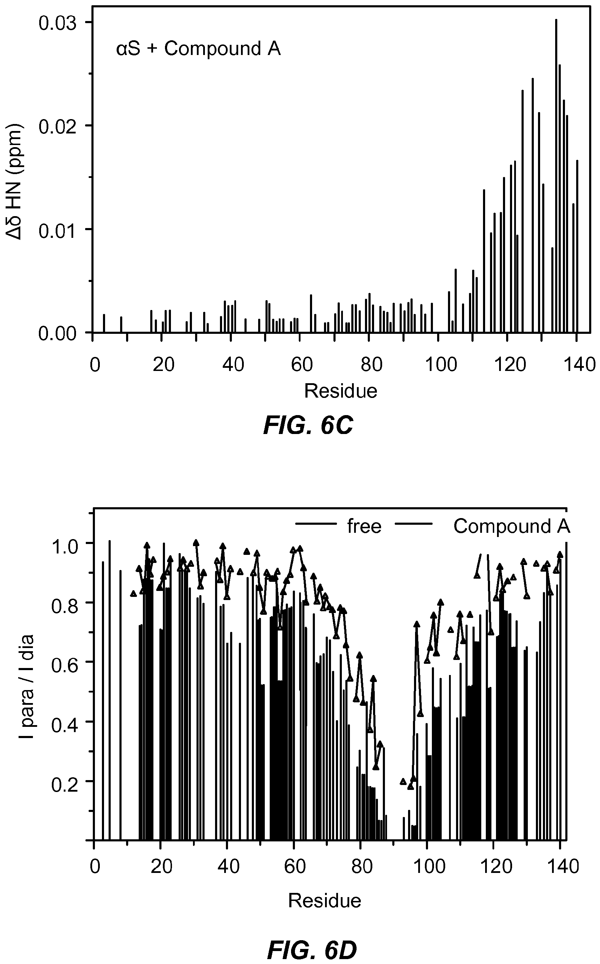

**Figure 6C** is a chemical shift perturbation analysis of 1H-15N resonances of alpha-synuclein in the presence of COMPOUND A at a 1:10 molar ratio.
**Figure 6D** is a profile of paramagnetic relaxation enhancement of amide protons in MTSL-labeled A90C alpha-synuclein (100 µM) in the absence (black) and presence (red) of COMPOUND A (3 mM).

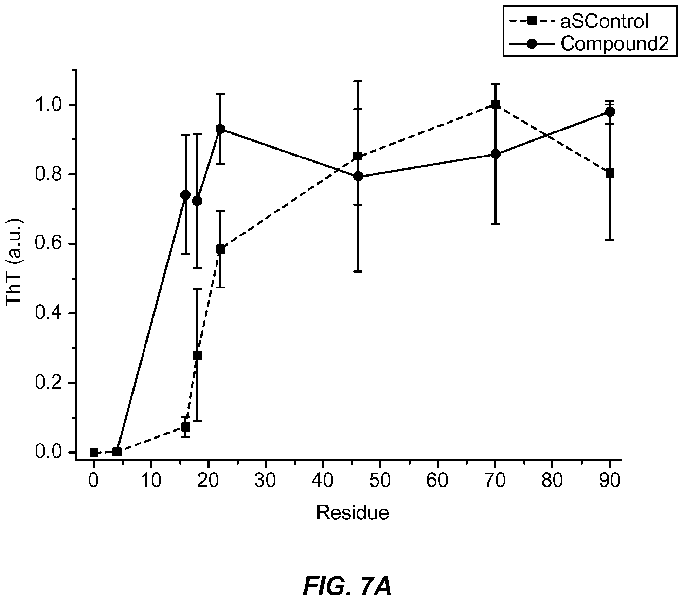

**Figure 7A** shows that the Thioflavin T assay indicates that COMPOUND A increases the rate of aggregation of alpha-synuclein *in vitro* by roughly two-fold.

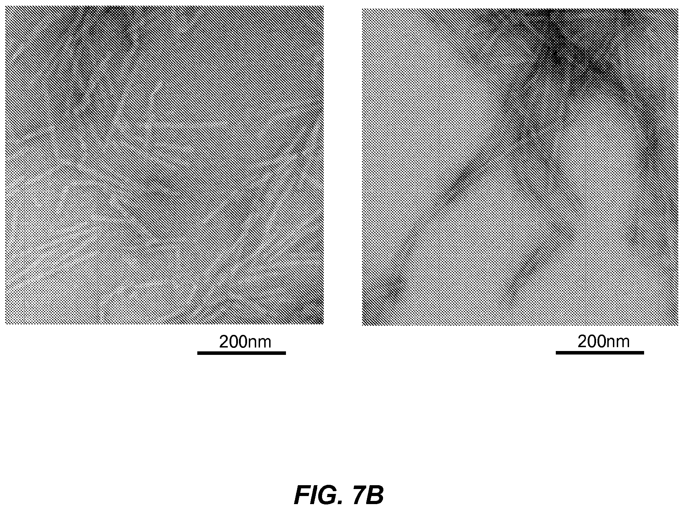

**Figure 7B** are electron micrographs of alpha-synuclein fibrils obtained in the presence (left) and absence (right) of COMPOUND A.

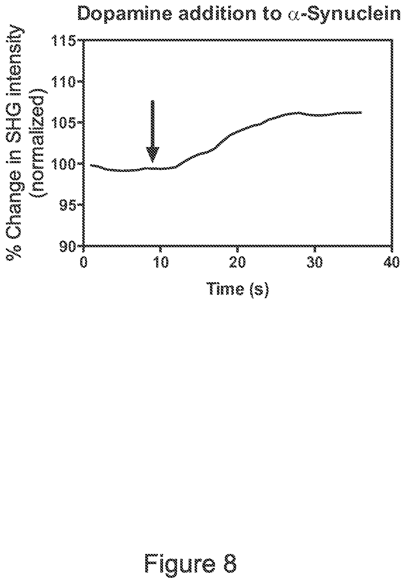

**Figure 8** shows kinetic (real time) change in SHG intensity of alpha-synuclein upon addition of dopamine.

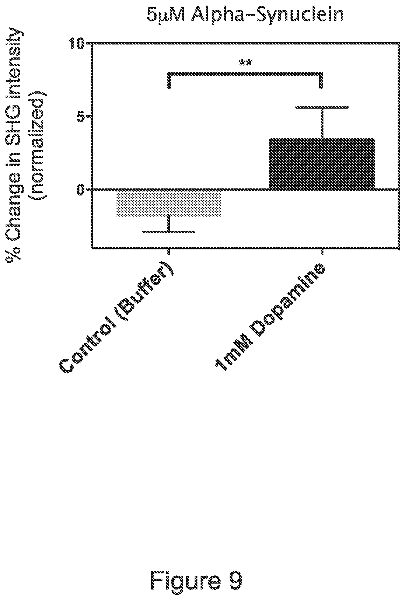

**Figure 9** is a quantification of an average change in SHG intensity of alpha-synuclein upon addition of either buffer alone or 1mM dopamine.

### DETAILED DESCRIPTION

### Definition

As used herein "second harmonic" refers to a frequency of light that is twice the frequency of a fundamental beam of light.

As used herein, "surface-selective" refers to a non-linear optical technique such as second harmonic generation or sum/difference frequency generation or other surface-specific technique known in the art.

As used herein, "sum frequency generation" (SFG) is a nonlinear, optical technique whereby light at one frequency (Ω₁) is mixed with light at another frequency (Ω₂) to yield a response at the sum frequency (Ω₁ + Ω₂) (Yang, G, Shen, YR, Optics Letters, 9(11): 510-512, 1984; Shen, YR, Annual Review of Physical Chemistry 40(1): 327-350, 1989). For example, SFG is particularly useful for the detection of molecules at surfaces through their characteristic vibrational transitions and, in this case, is essentially a surface-selective infrared spectroscopy with Ω₁ and Ω₂ at visible and infrared frequencies. When the terms "SHG" or "second harmonic generation" are used herein, it is understood that SFG and "sum frequency generation" can substitute and be used in place of SHG with methods well known to one skilled in the art.

A "nonlinear-active moiety," as used herein, is a substance which possesses a hyperpolarizability.

"Second harmonic-active moiety," as used herein, refers to a nonlinear-active moiety, particle or molecule which can be attached or incorporated (e.g., covalently or non-covalently attached, genetically incorporated, etc.) to a molecule (e.g., a protein, such as an enzyme), particle, cell or phase (e.g., lipid bilayer) in order to render it (e.g., more) nonlinearly optically active.

"Allosteric", "allosteric modulator", or "allosteric candidate" as used herein, refers to a molecule, moiety or substance which binds predominantly to a site other than the active site and may cause conformational change as determined by SHG or SFG (e.g., in one or more locations adjacent the binding site and/or in one or more locations not adjacent the binding site), and thus exert their effect via an allosteric mechanism of action.

"Active site" or "active binding site," as used herein, refers to a region of a target protein that, as a result of its shape and charge potential, favorably interacts or associates with another agent (e.g., a ligand, a protein, polypeptide, peptide, nucleic acid, including DNA or RNA, molecule, compound, antibiotic or drug, molecule, moiety, substrate, product, analog, or inhibitor), via various covalent and/or non-covalent binding forces and where function of the protein (e.g., catalysis, signaling, and/or effector activation) is performed.

The term "ligand", as defined herein includes any molecule that binds to another molecule, for example, one protein binding to another, a carbohydrate binding to a protein, or a small molecule binding to a protein.

As used herein, "nonlinear" refers to optical techniques capable of transforming the frequency of an incident light beam (a.k.a., the fundamental). The nonlinear beams are the beams (e.g., higher order frequency beams) which result from such a transformation, e.g., a second harmonic. In second harmonic, sum frequency or difference frequency generation, the nonlinear beams are generated coherently. In second harmonic generation (SHG), two photons of the fundamental beam are virtually scattered by the interface to produce one photon of the second harmonic. Also referred to herein as "nonlinear optical" or "surface-selective nonlinear."

The terms "nonlinear active" or "nonlinearly active" as used herein also refer to the general property of the ability of molecules, particles, an interface or a phase, to generate nonlinear optical radiation when driven by incident radiation beam or beams.

When referring herein to nonlinear optical methods, "detection" or "detecting" refers to those techniques by which the properties of surface-selective nonlinear optical radiation can be used to detect, measure or correlate properties of probe-target interactions (such as the interaction between a protein and a candidate compound), or effects of the interactions, with properties of the nonlinear optical light (e.g., intensity, wavelength, polarization or other property common to electromagnetic radiation).

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Introduction

Provided herein are methods for screening a candidate biochemical entity and/or a binding moiety (e.g., a binding moiety or moieties associated with the candidate biochemical entity) targeting a target biochemical entity, such as, for example, a candidate biochemical entity and/or binding moiety associated with an *in vivo* or *in vitro* pharmacological property. These methods are based on signals generated by a second harmonic generation (SHG)-active moiety, a sum frequency generation (SFG)-active moiety or a difference frequency generation (DFG)-active moiety (e.g., attached to the candidate, the target, or both) using a surface selective technique. The methods can involve comparing the signals of two or more candidates produced upon binding between the target and different candidates. In some cases, the candidates can be biochemical entities (e.g., molecules) having a same or similar scaffold core. In some cases, the biochemical entities may include drug candidates (and/or a binding moiety or moieties associated therewith) targeting a target biomolecule.

In some examples, the methods herein can be used for screening drug candidates (and/or a binding moiety associated therewith) targeting a target biomolecule, such as, for example, a candidate or binding moiety associated with an *in vivo* or *in vitro* pharmacological property. In some cases, the drug candidates can be molecules having a same or similar scaffold core. Any aspects of the disclosure described in relation to drug candidates (and/or a binding moiety associated therewith) and target biomolecules may equally apply to any type of candidate biochemical entity (and/or binding moiety) and any type of target biochemical entity at least in some configurations. For example, the present disclosure may be applied to cells. Further, any aspects of the disclosure described in relation to reference molecules may equally apply to any type of reference biochemical entity at least in some configurations.

It is generally believed that structural similarity leads to functional similarity to certain degrees. However, structural similarity alone does not always correlate to certain functions, particularly *in vivo* pharmacological properties. Therefore, drug candidates generated by structure-based design have to be screened extensively using various *in vivo* or *in vitro* assays to identify a lead compound. This process is tedious, time-consuming, expensive, and often futile, and has become one of the major bottlenecks in modern drug discovery.

In one aspect, the present disclosure provides a method of screening biochemical entities, the method comprising: (a) providing a library comprising a plurality of candidate biochemical entities, wherein the candidate biochemical entities are structurally similar as determined using one or more structural similarity parameters; (b) measuring a first conformational state of a target biochemical entity, thereby generating a baseline; (c) contacting each of the candidate biochemical entities with the target biochemical entity; (d) measuring a second conformational state of the target biochemical entity; (e) determining a signature of the target biochemical entity using the second conformational state; and (f) selecting one or more biochemical entities from the candidate biochemical entities based on the signature.

In another aspect, the present disclosure provides a method of screening biochemical entities, the method comprising: (a) providing a library comprising a plurality of candidate biochemical entities; (b) measuring a first conformational state of a target biochemical entity, thereby generating a baseline; (c) contacting each of the candidate biochemical entities with the target biochemical entity; (d) measuring a second conformational state of the target biochemical entity; (e) determining a signature for at least a first and a second candidate biochemical entity using the second conformational state; (f) comparing the signature of the first biochemical entity to the signature of the second biochemical entity; and (g) selecting, based on the comparison in (f), one or more biochemical entities from the candidate biochemical entities.

In a further aspect, the present disclosure provides a method of cataloging biochemical entities, the method comprising: (a) providing a library of biochemical entities, wherein each of the biochemical entities comprises a distinct molecular structure; (b) contacting at least one biochemical entity in the library with a same target biochemical entity; (c) measuring a conformational state of the target biochemical entity; (d) determining a signature using the conformational state; and (e) generating a structure-activity relationship (SAR) catalog using the signature.

In some cases, generating the SAR can comprise correlating the signature with a binding property, a functional property, an efficacy, a potency, a selectivity, or a combination thereof. In some cases, generating the SAR can comprise correlating the signature to one or more structural parameters of the candidate biochemical entities, wherein said structural parameters are selected from the group consisting of: molecular weight, chemical formula, number of total atoms, number of carbon atoms, number of hydrogen atoms, number of nitrogen atoms, number of oxygen atoms, number of sulfur atoms, polarity as measured by log D, polarity as measured by log P, polarity as measured by PSA (polar surface area), the scaffold core, chemical similarity as measured by graph theory, Tanimoto (or Jaccard) coefficient T, number of aromatic groups, number of heterocyclic groups, number of monocyclic groups, number of fused ring systems, and number of double bonds.

The method can further comprise analyzing the SAR to determine a binding moiety within at least one of the biochemical entities that is associated with a pharmacological property or a functional effect. In some cases, the conformational state of the target biochemical can be measured prior to the contacting to thereby generate a baseline.

The library can comprise 2, 3, 4, 5, 6, 7, 8, 9, 10, 11, 12, 13, 14, 15, 16, 17, 18, 19, 20, 22, 24, 26, 28, 30, 35, 40, 45, 50, 60, 70, 80, 90, 100, 120, 140, 160, 180, 200, 250, 300, 350, 400, 450, 500, 600, 700, 800, 900, 1000, 1200, 1400, 1600, 1800, 2000, 2500, 3000, 3500, 4000, 4500, 5000, 6000, 7000, 8000, 9000, or 1000, or more candidate biochemical entities. In some examples, the library can comprise 6 or more candidate biochemical entities. In further examples, the library can comprise 20 or more candidate biochemical entities. Additionally, the library of biochemical entities can comprise drug candidates.

The candidate biochemical entities can be structurally similar as determined using 1, 2, 3, 4, 5, 6, 7, 8, 9, 10, 11, 12, 13, 14, 15, 16, 17, 18, 19, 20, or more structural similarity parameters. In some examples, the candidate biochemical entities can be structurally similar as determined using 3 or more structural similarity parameters. In further examples, the candidate biochemical entities can be structurally similar as determined using 5 or more structural similarity parameters. Examples of structural similarity parameters include but are not limited to:
(i) molecular weight within 10, 20, 30, 40, 50, 60, 70, 80, 90, 100, 110, 120, 130, 140, 150, 160, 170, 180, 190, 200, 210, 220, 230, 240, 250, 260, 270, 280, 290, 300 g/mol of each of the candidate biochemical entities;
(ii) molecular weight within 1%, 2%, 3%, 4%, 5%, 6%, 7%, 8%, 9%, 10%, 11%, 12%, 13%, 14%, 15%, 16%, 17%, 18%, 19%, 20,%, 22% 24%, 26%, 28%, 30%, 35%, 40%, 45% or 50% of each of the candidate biochemical entities
(iii) chemical formula to within 1, 2, 3, 4, 5, 6, 7, 8, 9, 10, 11, 12, 13, 14, 15, 16, 17, 18, 19, 20, 22, 24, 26, 28, 30, 35, 40, 45, 50, 60, 70, 80, 90, 100, 120, 140, 160, 180, 200, 250, 300, 350, 400, 450, 500, 600, 700, 800, 900, or 1000 atoms of each of the candidate biochemical entities;
(iv) a difference in total number of atoms of less than 1, 2, 3, 4, 5, 6, 7, 8, 9, 10, 11, 12, 13, 14, 15, 16, 17, 18, 19, 20, 22, 24, 26, 28, 30, 35, 40, 45, 50, 60, 70, 80, 90, 100, 120, 140, 160, 180, 200, 250, 300, 350, 400, 450, 500, 600, 700, 800, 900, or 1000 between each of the candidate biochemical entities;
(v) a difference in number of carbon atoms of less than 1, 2, 3, 4, 5, 6, 7, 8, 9, 10, 11, 12, 13, 14, 15, 16, 17, 18, 19, or 20;
(vi) a difference in number of hydrogen atoms of less than 1, 2, 3, 4, 5, 6, 7, 8, 9, 10, 11, 12, 13, 14, 15, 16, 17, 18, 19, or 20;
(vii) a difference in number of nitrogen atoms of less than 1, 2, 3, 4, 5, 6, 7, 8, 9, 10, 11, 12, 13, 14, 15, 16, 17, 18, 19, or 20;
(viii) a difference in number of oxygen atoms of less than 1, 2, 3, 4, 5, 6, 7, 8, 9, 10, 11, 12, 13, 14, 15, 16, 17, 18, 19, or 20;
(ix) a difference in number of sulfur atoms of less than 1, 2, 3, 4, 5, 6, 7, 8, 9, 10, 11, 12, 13, 14, 15, 16, 17, 18, 19, or 20;
(x) a polarity as measured by log D within 1%, 2%, 3%, 4%, 5%, 6%, 7%, 8%, 9%, 10%, 11%, 12%, 13%, 14%, 15%, 16%, 17%, 18%, 19%, 20,%, 22% 24%, 26%, 28%, 30%, 35%, 40%, 45% or 50% of each of the candidate biochemical entities;
(xi) a polarity as measured by log P (partition coefficient) within 1%, 2%, 3%, 4%, 5%, 6%, 7%, 8%, 9%, 10%, 11%, 12%, 13%, 14%, 15%, 16%, 17%, 18%, 19%, 20,%, 22% 24%, 26%, 28%, 30%, 35%, 40%, 45% or 50% of each of the candidate biochemical entities;
(xii) a polarity as measured by PSA (polar surface area) within 1%, 2%, 3%, 4%, 5%, 6%, 7%, 8%, 9%, 10%, 11%, 12%, 13%, 14%, 15%, 16%, 17%, 18%, 19%, 20,%, 22% 24%, 26%, 28%, 30%, 35%, 40%, 45% or 50% of each of the candidate biochemical entities;
(xiii) an identical scaffold core;
(xiv) a chemical similarity as measured by graph theory;
(xv) a Tanimoto (or Jaccard) coefficient T greater than 0.5, 0.6, 0.7, 0.75, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, or 0.99;
(xvi) a difference in number of aromatic groups of less than 1, 2, 3, 4, 5, 6, 7, 8, 9, or 10;
(xvii) a difference in number of heterocyclic groups of less than 1, 2, 3, 4, 5, 6, 7, 8, 9, or 10;
(xviii) a difference in number of monocyclic groups of less than 1, 2, 3, 4, 5, 6, 7, 8, 9, or 10;
(xix) a difference in number of fused ring systems of less than 1, 2, 3, 4, 5, 6, 7, 8, 9, or 10;
   and
(xx) a difference in number of double bonds of less than 1, 2, 3, 4, 5, 6, 7, 8, 9, or 10.

In some cases, the target biochemical entity can be an intrinsically disordered protein. For example, the intrinsically disordered protein can be alpha-synuclein. In some cases, the target biochemical entity can be a drug target. In some cases, the target biochemical entity can have no known conformational modulators.

In some cases, the first conformational state can be measured after the target biochemical entity is incubated with an agent. For example, the agent can be a candidate biochemical entity from the library

In some cases, the conformational state(s) can be measured using total internal reflection (TIR). In some cases, the conformational state(s) can be measured using second harmonic generation (SHG).

In some cases, determining the signature can comprise measuring a kinetic (real time) change in conformational state of the target biochemical entity. In other cases, determining the signature can comprise measuring an end-point change in conformational state of the target biochemical entity. In yet other cases, determining the signature can comprise measuring a signal intensity of the signature. In further cases, the determining of the signature can use the baseline. Additionally, the signature can indicate a change in the conformational state of the target biochemical entity.

In some cases, the candidate biochemical entities can be analyzed based on one or more structural similarity parameters using a computer-executable program. In other cases, the signature can be analyzed using a computer-executable program.

Each of the candidate biochemical entities can produce a different change in conformational state of the target biochemical entity. Further, each of the candidate biochemical entities can generate a distinct signature.

In some cases, the change in conformational state of the target biochemical entity can be further compared to a change in conformational state produced by a known conformational modulator. In some cases, the known conformational modulator can be a known drug. In some cases, the biochemical entities can be selected based on the change in conformational state that is most similar to the change in conformational state induced by the known conformational modulator. In addition, the change in conformational state produced by the known conformational modulator can correlate to a pharmacological property or a functional effect.

In some cases, the signature or the SAR catalog of one or more of the biochemical entities can correlate to a known pharmacological property or a known functional effect. The one or more biochemical entities can be further selected based on the correlation of the signature or the SAR catalog to a known pharmacological property or a known functional effect.

In some cases, at least 2, 3, 4, 5, 6, 7, 8, 9, 10, 11, 12, 13, 14, 15, 16, 17, 18, 19, 20, 22, 24, 26, 28, 30, 35, 40, 45, 50, 60, 70, 80, 90, or 100 of the candidate biochemical entities have a similar pharmacological property or functional effect. In other cases, at least 2, 3, 4, 5, 6, 7, 8, 9, 10, 11, 12, 13, 14, 15, 16, 17, 18, 19, 20, 22, 24, 26, 28, 30, 35, 40, 45, 50, 60, 70, 80, 90, or 100 of the candidate biochemical entities do not have a similar pharmacological property or functional effect.

The methods provided in the present disclosure can further comprise measuring a signature parameter. Examples of signature parameters include but are not limited to:
i) a change in magnitude of the signature relative to the baseline;
ii) a change in direction of the signature relative to the baseline; and
iii) a change in rate of a signal per unit time of the signature upon the contacting of the candidate biochemical entity.

In some cases, the signature generated by a first candidate biochemical entity and the signature generated by a second candidate biochemical entity can be similar as determined using 1, 2, 3, or more signature similarity parameters. Examples of signature similarity parameters include but are not limited to:
i) the change in magnitude of the signature relative to the baseline upon contacting the first candidate biochemical entity is within 1%, 2%, 3%, 4%, 5%, 6%, 7%, 8%, 9%, 10%, 11%, 12%, 13%, 14%, 15%, 16%, 17%, 18%, 19%, 20,%, 22% 24%, 26%, 28%, 30%, 35%, 40%, 45%, or 50% of the change in magnitude of the signature relative to the baseline upon contacting the second candidate biochemical entity;
ii) the change in direction of the signature relative to the baseline upon contacting the first candidate biochemical entity is the same as the change in direction of the signature relative to the baseline upon contacting the second candidate biochemical entity; and
iii) the change in rate of a signal per unit time of the signature upon contacting the first candidate biochemical entity is within 1%, 2%, 3%, 4%, 5%, 6%, 7%, 8%, 9%, 10%, 11%, 12%, 13%, 14%, 15%, 16%, 17%, 18%, 19%, 20,%, 22% 24%, 26%, 28%, 30%, 35%, 40%, 45%, 50%, 60%, 70%, 80%, 90%, 100% (one-fold), 110%, 120%, 130%, 140%, 150%, 160%, 170%, 180%, 190%, or 200% (two-fold) of the change in rate of a signal per unit time of the signature upon contacting the second candidate biochemical entity.

As detailed in the examples below, the present disclosure provides methods for correlating SHG signals (or SFG signals, DFG signals) generated by a candidate to its *in vivo* or *in vitro* pharmacological properties, particularly its *in vivo* properties. Thus, the present disclosure provides methods of efficiently screening for drug candidates having one or more desired *in vivo* or *in vitro* pharmacological properties, obviating the need for expensive *in vivo* or *in vitro* assays for every candidate.

### I. Methods of screening drug candidates targeting a target molecule

Provided herein are methods for screening drug candidates targeting a target biomolecule. The methods are useful for screening any drug candidates, such as, for example, drug candidates having a same or similar scaffold core targeting a binding site in the target biomolecule.

### A. Drug candidates having a same or similar scaffold

In the context of small molecule drugs, "scaffold" can refer to a small target binding molecule to which one or more additional chemical moieties can be covalently attached, modified, or eliminated to form a plurality of molecules with common structural elements. Exemplary moieties include a halogen atom, a hydroxyl group, a methyl group, a nitro group, a carboxyl group, or any other type of molecular group. Characteristics of a scaffold can include, for example, binding at a target molecule binding site such that one or more substituents on the scaffold are situated in binding pockets in the target molecule binding site; having chemically tractable structures that can be chemically modified, particularly by synthetic reactions, so that a combinatorial library can be easily constructed; having chemical positions where moieties can be attached that do not interfere with binding of the scaffold to a protein binding site, such that the scaffold or library members can be modified to form ligands, to achieve additional desirable characteristics, e.g., enabling the ligand to be actively transported into cells and/or to specific organs, or enabling the ligand to be attached to a chromatography column for additional analysis. Thus, a molecular scaffold can be a small, identified target-binding molecule prior to modification to improve *in vivo* or *in vitro* pharmacological properties (e.g., binding affinity and/or specificity).

In the context of small molecule drugs, "scaffold core" can refer to the core structure of a molecular scaffold onto which various substituents can be attached. Thus, for a number of scaffold molecules of a particular chemical class, the scaffold core is common to all the scaffold molecules. In many cases, the scaffold core can consist of or include one or more ring structures. A scaffold group refers to a set of compounds that share a scaffold core and thus can all be regarded as derivatives of one scaffold molecule.

In the context of biologics, "scaffold" can refer to a set (e.g., a minimal set) of amino acid residues or nucleotides that allow a biological drug to interact with another molecule (e.g., a protein, a DNA, a RNA, a metal, a peptide, a polysaccharide). For example, the scaffold can comprise amino acid residues or nucleotides that have a specific geometric shape inherent to the motif. This geometric shape encompasses distance between the amino acid residues, angles between amino acid residues and the orientation of the amino acid residues.

In the context of biologics, "scaffold core" can refer to a set of consensus amino acid residues or nucleotides common to all the scaffold molecules. Substitutions such as mutations, deletions, insertions can be made to the scaffold core. Thus, for a number of scaffold molecules of a particular biologics class, the scaffold core is common to all the scaffold molecules. A scaffold group refers to a set of biologics that share a scaffold core and thus can all be regarded as derivatives of one scaffold molecule. The biologics molecules within a scaffold group can share 20%, 30%, 40%, 50%, 60%, 70%, 80%, 85%, 90%, 95%, 98%, 99%, or more sequence homology.

Scaffold or scaffold core can target a binding site in a target biomolecule. The binding site can be an active binding site or an allosteric binding site. Generally speaking, a binding site is an area of a target molecule to which a ligand can bind (covalently or non-covalently). A binding site embodies particular shapes and can contain one or more binding pockets. The particular shapes are often conserved within a class of target biomolecules, such as a molecular family. Binding sites within a class also can contain conserved structures such as, for example, moieties, the presence of a binding pocket, and/or an electrostatic charge at the binding site or some portion of the binding site, all of which can influence the shape of the binding site. A binding pocket refers to a specific volume within a binding site. A binding pocket is a particular space within a binding site at least partially bounded by target molecule atoms. Thus a binding pocket is a particular shape, indentation, or cavity in the binding site. Binding pockets can contain particular chemical groups or structures that are important in the non-covalent binding of another molecule such as, for example, groups that contribute to ionic, hydrogen bonding, van der Waals, or hydrophobic interactions between the molecules.

The methods provided herein can be used to screen any drug candidates, including small molecules and biologics (e.g., proteins, peptides, nucleic acids, antibodies). For example, the methods are useful for screening small molecule or antibody drugs targeting alpha synuclein. In some methods, a lead compound or a lead biologics is identified. A series of candidate compounds or biologics can be designed and selected based on the lead compound or lead biologics. For example, computer modeling methods can be used to generate candidate compounds or biologics.

In some cases, a candidate compound or biologics can be docked into a computer-generated binding pocket of the target biomolecule. In some computer modeling methods, the candidate can be superimposed to the experimentally determined disposition of a known lead compound or biologics of the target biomolecule, e.g., a crystal structure of the target biomolecule-agonist or the target biomolecule-antagonist co-crystal. Molecular optimization techniques can be used to predict a degree of structural fit, as well as the energy of interaction, of the candidate and the target biomolecule (or its segment). The degree of structural fit determines how well a candidate small molecule can physically occupy the space in the milieu of the binding pocket; surface contours of the target biomolecule (and the candidate) can be constructed in various ways for this purpose; for example van der Waals radii of atoms are used to construct the surface. Energy of interaction can be computed by ab initio methods (such as density functional theory methods) or by empirical methods (such as molecular mechanics methods). The energy of interaction can also be computed by a hybrid of the above methods. Molecular optimization techniques also allow for molecular flexibility, which indicates that changes in bond length, bond angles and bond torsions are allowed.

Structural information for the target biomolecule (or the appropriate portions of target biomolecule) can be obtained directly by X-ray crystallography, indirectly by homology modeling, or a combination thereof. Homology modeling may also be performed to obtain the structure of only a portion of the target biomolecule. This is typically achieved by using an analogous portion of one of the known structures as the template. Various methods and software may be used for this purpose; for example, the methods and software described in SWISS-MODEL: an automated protein homology-modeling server (Schwede, T. ; Kopp, J.; Guex, N.; Peitsch, M. Nucleic Acids Research. (2003) 31(13):3381-3385) may be used. Software and methods as taught by U.S. Patent 7,384,773, and the references therein, may also be used for modeling.

A number of programs may be used to process the machine-readable data. For example, the computer program DOCK and variants thereof is widely used (Lorber, D. and Shoichet, B., Protein Science, 7:938-950, 1998). Other exemplary programs include Autodock (Scripps Research Institute, La Jolla, Calif.), QUANTA (Molecular Simulations, San Diego, Calif.), Insight (Molecular Simulations, San Diego, Calif.), SYBYL (TRIPOS, Inc., St. Louis, Mo.) and LEAPFROG (TRIPOS, Inc., St. Louis, Mo.).

A "library" can refer to a group of biochemical entities (e.g. candidate biochemical entities) used in the methods of the present disclosure. For example, a library of biochemical entities can comprise 2, 3, 4, 5, 6, 7, 8, 9, 10, 11, 12, 13, 14, 15, 16, 17, 18, 19, 20, 22, 24, 26, 28, 30, 35, 40, 45, 50, 55, 60, 65, 70, 75, 80, 85, 90, 95, 100, 110, 120, 130, 140, 150, 160, 170, 180, 190, 200, 220, 240, 260, 280, 300, 350, 400, 450, 500, 550, 600, 650, 700, 750, 800, 850, 900, 950, 1000, 1100, 1200, 1300, 1400, 1500, 1600, 1700, 1800, 1900, 2000, 2200, 2400, 2600, 2800, 3000, 3200, 3400, 3600, 3800, 4000, 4500, 5000, 6000, 7000, 8000, 9000, or 10000, more biochemical entities.

The biochemical entities can be structurally similar. Two or more biochemical entities can be determined to be structurally similar based on one or more structural similarity parameters including but not limited to: molecular weight; chemical formula; number of total atoms; number of specific atoms (e.g. carbon, hydrogen, nitrogen, oxygen, and/or sulfur); polarity; scaffold core; chemical stability as measured by graph theory; Tanimoto (or Jaccard) coefficient, T; number of aromatic groups, heterocyclic group, monocyclic group and/or fused ring systems; or number of double bonds. For example, two or more biochemical entities can be determined to be structurally similar if the molecular weights of the two or more biochemical entities are within 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 600, 700, 800, 900, or 1000 g/mol of each other. Alternatively, two or more biochemical entities can be determined to be structurally similar if the molecular weights of the two or more biochemical entities are within 5%, 10%, 15%, 20%, 25%, 30%, 35%, 40%, 45%, or 50% of each other. In some cases, two or more biochemical entities can be determined to be structurally similar if the chemical formulae of each of the two or more biochemical entities are within 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 atoms of each other. In some cases, two or more biochemical entities can be determined to be structurally similar if the difference in total number of atoms between each of the two or more biochemical entities is less than 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30. In further cases, two or more biochemical entities can be determined to be structurally similar if the difference in number of one or more specific atoms (e.g. carbon, hydrogen, nitrogen, oxygen and/or sulfur atoms) between each of the two or more biochemical entities is less than 1, 2, 3, 4, 5, 6, 7, 8, 9, 10, 11, 12, 13, 14, 15, 16, 17, 18, 19, or 20. For example, two or more biochemical entities can be determined to be structurally similar if the difference in number of carbon atoms is less than 5 and/or the difference in number of oxygen atoms is less than 3. In some cases, two or more biochemical entities can be determined to be structurally similar if the polarity [e.g. as measured by log D, log P (partition coefficient), or PSA (polar surface area)] of the two or more biochemical entities are within 1%, 2%, 3%, 4%, 5%, 6%, 7%, 8%, 9%, 10%, 12%, 12%, 14%, 16%, 18%, 20%, 25%, 30%, 35%, 40%, 45%, or 50% of each other. In some cases, two or more biochemical entities can be determined to be structurally similar if the two or more biochemical entities have a same scaffold core (as defined above). In other cases, two or more biochemical entities can be determined to be structurally similar if the two or more biochemical entities exhibit a chemical similarity as measured by graph theory. In further cases, two or more biochemical entities can be determined to be structurally similar if the two or more biochemical entities exhibit a Tanimoto (or Jaccard) coefficient T greater than 0.50, 0.55, 0.60, 0.65, 0.70, 0.75, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, or 0.99. In some cases, two or more biochemical entities can be determined to be structurally similar if the difference in number of aromatic groups, heterocyclic groups, monocyclic groups and/or fused ring systems in each of the two or more biochemical entities is less than 1, 2, 3, 4, 5, 6, 7, 8, 9, or 10. In some cases, two or more biochemical entities can be determined to be structurally similar if the difference in number of double bonds in each of the two or more biochemical entities is less than 1, 2, 3, 4, 5, 6, 7, 8, 9, or 10.

Further, two or more biochemical entities can be determined by chemical analysis software. Examples of chemical analysis software include but are not limited to Spinitex, Toxmatch, and Simfinder.

### B. Selecting drug candidates

Drug candidates, such as, for example, drug candidates having a same or similar scaffold core can be screened using the methods of the disclosure. These methods are based on signals generated by a SHG moiety, a SFG moiety or a DFG moiety (e.g., attached to the candidate, the target, or both) using a surface selective technique. The signal is produced and detected upon binding between the target and a candidate molecule.

It was surprisingly discovered in the present invention that, despite structural similarity among different drug candidates sharing a same or similar scaffold, these drug candidates can generate signals having varying degree of differences. In some cases, as detailed in the examples below, two structurally closely related candidates can generate starkly different signals whereas two structurally dissimilar molecules can produce very similar signals. More surprisingly, it was discovered in the present invention that the detected signal difference correlates to a difference in an *in vivo* or *in vitro* pharmacological property. Accordingly, the methods of the invention can be used to screen compounds and biologics having one or more desired *in vivo* or *in vitro* pharmacological properties. Drug candidates can be screened according to various embodiments of the invention.

### 1. Direct comparison

In some examples, the signals generated from two or more molecules are compared to each other. The comparison can be made between or among drug candidates from a chemical library, or drug candidates generated and selected from, for example, the computer modeling methods described herein. The comparison can also be made between a drug candidate and a reference molecule. A reference molecule can be a known drug, or a lead compound, or otherwise a molecule associated with an *in vivo* or *in vitro* pharmacological property (e.g., a desired property). The reference molecule and the drug candidates can have a same or similar scaffold core targeting a binding site in the target biomolecule.

In some methods, the target biomolecule is contacted in parallel with two or more candidates, e.g., two candidates having a same or similar scaffold core targeting a binding site in the target biomolecule. A signal is produced upon binding between the target biomolecule and each candidate. The signals produced by different candidates are quantified and compared to each other. The difference between signals produced by different candidates can be generated. The difference can be a positive difference or a negative difference. In some cases, a positive difference correlates to an improvement in the *in vivo* or *in vitro* pharmacological property. In some cases, a positive difference correlates to a decrease in the *in vivo* or *in vitro* pharmacological property. In other cases, a negative difference correlates to an improvement in the *in vivo* or *in vitro* pharmacological property. In some other cases, a negative difference correlates to a decrease in the *in vivo* or *in vitro* pharmacological property.

Because the difference indicates a difference in an *in vivo* or *in vitro* pharmacological property, a drug candidate can be selected based on this difference. For example, in cases where a positive difference correlates to an improvement in the *in vivo* or *in vitro* pharmacological property, the candidate having the higher signal is selected. In cases where a negative difference correlates to an improvement in the *in vivo* or *in vitro* pharmacological property, the candidate having the lower signal is selected.

Likewise, a drug candidate can be selected simply by ranking the signals generated by different drug candidates. In cases where an increase in signal is correlated to an improvement in the *in vivo* or *in vitro* pharmacological property, the candidate having a higher signal is selected. In cases where a decrease in signal is correlated to an improvement in the *in vivo* or *in vitro* pharmacological property, the candidate having a lower signal is selected.

Signals for different candidates and the reference molecule can be concentration-normalized. In some methods, a signal difference 1%, 5%, 10%, 20%, 30%, 40%, 50%, 100%, 200%, 300%, 400%, 500% or more above noise level indicates a difference in an *in vivo* or *in vitro* pharmacological property.

### 2. Pre-incubation with a candidate molecule

In some examples, signals from a drug candidate and a reference molecule are indirectly compared. A reference molecule can be a known drug, or a lead compound, or otherwise a molecule associated with an *in vivo* or *in vitro* pharmacological property (e.g., a desired property). The reference molecule can target the same binding site as the candidate molecule. The reference molecule and the drug candidate can have a same or similar scaffold core targeting a binding site in the target biomolecule.

Specifically, a target biomolecule is first contacted with a drug candidate, forming a binding complex of the target biomolecule and a candidate molecule. The binding complex is then contacted with the reference molecule. As detailed in the examples below, a signal produced upon contacting the binding complex with the reference molecule correlates with *in vivo* or *in vitro* pharmacological property of the molecules. If the candidate molecule has a decreased *in vivo* or *in vitro* pharmacological property as compared to the reference molecule, a signal can be detected upon contacting the binding complex with the reference molecule. If the candidate molecule has an increased *in vivo* or *in vitro* pharmacological property as compared to the reference molecule, no signal or only a small signal can be detected. Therefore, candidate molecules can be screened and selected based on this correlation, i.e., no signal or a small signal indicates an increased or at least comparable *in vivo* or *in vitro* pharmacological property as compared to the reference molecule.

Signals for different candidates and the reference molecule can be concentration-normalized. The signal can be a positive signal or a negative signal. In some methods, a signal of no more than 1%, 5%, 10%, 20%, 30%, 40%, 50%, 100%, or 200%, above noise level indicates an increased or at least comparable *in vivo* or *in vitro* pharmacological property as compared to the reference molecule. In some examples, a signal of no more than 1%, 5% or 10% is used as a cutoff value for selecting or identifying candidates having an increased or at least comparable *in vivo* or *in vitro* pharmacological property as compared to the reference molecule.

### 3. Pre-incubation with a reference molecule

In some examples, signals from a drug candidate and a reference molecule are indirectly compared. A reference molecule can be a known drug, or a lead compound, or otherwise a molecule associated with an *in vivo* or *in vitro* pharmacological property (e.g., a desired property). The reference molecule can target the same binding site as the candidate molecule. The reference molecule and the drug candidate can have a same or similar scaffold core targeting a binding site in the target biomolecule.

Specifically, a target biomolecule is first contacted with a reference candidate, forming a binding complex of the target biomolecule and a reference molecule. The binding complex is then contacted with a candidate molecule. If the candidate molecule has a decreased *in vivo* or *in vitro* pharmacological property as compared to the reference molecule, no signal or only a small signal can be detected. If the candidate molecule has an increased *in vivo* or *in vitro* pharmacological property as compared to the reference molecule, a signal can be detected upon contacting the binding complex with the reference molecule. Therefore, candidate molecules can be screened and selected based on this correlation, i.e., a signal indicates an increased or at least comparable *in vivo* or *in vitro* pharmacological property as compared to the reference molecule.

Signals for different candidates and the reference molecule can be concentration-normalized. The signal can be a positive signal or a negative signal. In some methods, a signal of more than 1%, 5%, 10%, 20%, 30%, 40%, 50%, 100%, or 200%, above noise level indicates an increased or at least comparable *in vivo* or *in vitro* pharmacological property as compared to the reference molecule. In some examples, a signal of more than 1%, 5% or 10% is used as a cutoff value for selecting or identifying candidates having an increased or at least comparable *in vivo* or *in vitro* pharmacological property as compared to the reference molecule.

### A. Other assays

Once a drug candidate having a desired property is identified using the methods described herein, the property can be confirmed by one or more *in vivo* or *in vitro* assays.

*In vitro* assays can include, but are not limited to, crystallography, nuclear magnetic resonance spectroscopy, mass spectroscopy, and cellular assays. General principles of cellular assays are well known in the art, and include methods in which a drug candidate is delivered into the cell (e.g., by electroporation, passive diffusion, microinjection and the like) and an activity endpoint is measured, such as changes in the cellular phenotype known to be affected by the activity of the particular drug candidate being measured.

*In vivo* assays include screening a drug candidate in animal models (e.g., whole animals or animal organs). Thus, the drug candidate may be further tested in cell models or animal models for their ability to cause detectable changes in phenotype related to a particular activity. In addition to cell cultures, animal models may be used to test the drug candidate for its ability to treat diseases in an animal model.

### B. In vivo or in vitro pharmacological property

As discussed above, the detected signal or difference in signal can be used to determine an *in vivo* or *in vitro* pharmacological property. In some cases, the pharmacologic property may include a pharmacokinetic property. Relevant *in vitro* pharmacological properties that can be screened using the methods described herein include, but are not limited to, binding affinity, binding specificity, and binding avidity. Relevant *in vivo* pharmacological properties that can be screened using the methods described herein include, but are not limited to, therapeutic efficacy, pharmacokinetics properties, preclinical toxicity properties, clinical efficacy properties, clinical toxicity properties, and clinical safety properties.

The detected signal or difference in signal can be used to determine a functional effect. For example, the detected signal or difference in signal can correlate to a functional phenotype (e.g., *in vivo*), mechanism of action (e.g., *in vivo* or *in vitro*), or a combination thereof. In some examples, a difference in the detected signal can result from a comparison of a signal produced by a drug candidate with a signal produced by a known drug, wherein the drug candidate's functional effects (e.g. functional phenotype, mechanism of action) can be determined based on a similarity in detected signal (e.g. SHG signature, SFG, signature, or DFG signature with that of the known drug. In some cases, an *in vitro* and/or *in vivo* pharmacological property can be associated with or related to the functional effect, or vice versa. For example, a therapeutic efficacy, a binding affinity or any other *in vitro* or *in vivo* pharmacological property can be associated with a mechanism of action determined using the methods described herein. Thus, any aspects of the disclosure described in relation to screening *in vitro* or *in vivo* pharmacological properties may equally apply to screening *in vitro* or *in vivo* functional phenotype and/or mechanism of action at least in some configurations.

Therapeutic efficacy refers to the therapeutic effect of a drug or candidate drug in a subject (e.g., a human being, an animal). Therapeutic efficacy is the ability of the drug or candidate drug to (a) prevent the development of a disease state or pathological condition, either by reducing the likelihood of or delaying onset of the disease state or pathological condition or (b) reduce or eliminate some or all of the clinical symptoms associated with the disease state or pathological condition.

Relevant pharmacokinetics properties include bioavailability (i.e., fraction of the dose which reaches the systemic circulation as intact drug), fraction unbound (i.e., the extent to which a drug is bound in plasma or blood; [unbound drug concentration]/[total drug concentration]), fraction of drug that is metabolized by drug metabolizing enzyme, total area under the plasma drug concentration-time curve, and the volume of blood cleared of drug per unit time.

Relevant preclinical toxicity properties include 50% therapeutic dose (TD50; the dose of the medication that results in 50% of the animals tested achieving the desired therapeutic outcome), 50% lethal dose (LD50; the dose of the medication that results in 50% of the animals tested dying), and therapeutic index (the ratio of LD50/TD50; a measure of the drug's inherent toxicity).

Relevant clinical efficacy properties include the number of patients who need to be treated to reach 1 desired outcome, and odds ratio (the odds of reaching the desired outcome among the treated cases compared with the odds of not reaching the desired outcome among the controls).

Relevant clinical toxicity properties include the number of patients who need to be treated to have 1 toxicity outcome, and odds ratio (the odds of reaching the drug toxicity among the treated cases compared with the odds of not reaching drug toxicity among the controls).

In some cases, the detected signal or signal difference correlates to not only *in vitro* but also *in vivo* pharmacological properties. In some cases, the detected signal or signal difference correlates to *in vivo* pharmacological properties, but does not correlate to *in vitro* pharmacological properties. In some cases, the detected signal or signal difference correlates to *in vitro* pharmacological properties, but does not correlate to *in vivo* pharmacological properties.

In some cases, the detected signal or signal difference correlates to more than one *in vivo* or *in vitro* pharmacological properties. For example, in some cases, the detected signal or signal difference correlates not only to binding affinity, but also to other properties such as therapeutic efficacy, pharmacokinetics properties, preclinical toxicity, clinical efficacy, or clinical toxicity.

In some cases, the detected signal or signal difference correlates to a difference in some *in vivo* or *in vitro* pharmacological properties but not to a difference in other *in vivo* or *in vitro* pharmacological properties. For example, in some cases, the detected signal or signal difference correlates to therapeutic efficacy, pharmacokinetics properties, preclinical toxicity, clinical efficacy, or clinical toxicity, but does not correlate to binding affinity, binding specificity, or binding avidity.

### II. Methods of identifying a binding moiety for a target biomolecule

Provided herein are methods for identifying a binding moiety for a target biomolecule, such as, for example, a binding moiety that is associated with an *in vivo* or *in vitro* pharmacological property. The methods are based on signals generated by a SHG-active moiety, a SFG-active moiety, or a DFG-active moiety (e.g., attached to the candidate or the target) using a surface selective technique. Typically, the methods involve comparing the structures of two or more candidates, such as, for example, candidates having a same or similar scaffold core. The identification is based on signal difference between signals produced and detected upon binding between the target and different candidates.

As discussed above, it was surprisingly discovered that the signal difference correlates to a difference in an *in vivo* or *in vitro* pharmacological property. Thus, the binding moiety identified using the methods described herein is not merely just an additional binding moiety. Rather, the identified binding moiety is a functionally relevant moiety that enhances one or more desired *in vivo* or *in vitro* pharmacological properties of a candidate.

In some cases, the identified binding moiety correlates to not only *in vitro* but also *in vivo* pharmacological properties. In some cases, the identified binding moiety correlates to *in vivo* pharmacological properties, but does not correlates to *in vitro* pharmacological properties. In some cases, the identified binding moiety correlates to *in vitro* pharmacological properties, but does not correlates to *in vivo* pharmacological properties.

In some cases, the identified binding moiety correlates to more than one *in vivo* or *in vitro* pharmacological properties. For example, in some cases, the identified binding moiety correlates not only to binding affinity, but also to other properties such as therapeutic efficacy, pharmacokinetics properties, preclinical toxicity, clinical efficacy, or clinical toxicity.

In some cases, the identified binding moiety correlates to a difference in some *in vivo* or *in vitro* pharmacological properties but not to a difference in other *in vivo* or *in vitro* pharmacological properties. For example, in some cases, the identified binding moiety correlates to therapeutic efficacy, pharmacokinetics properties, preclinical toxicity, clinical efficacy, or clinical toxicity, but does not correlate to binding affinity, binding specificity, or binding avidity.

The signal difference between molecules can be measured according to various methods as described above in connection with the methods for screening drug candidates targeting a target biomolecule. For example, the signal difference between molecules can be measured by directly comparing the signals generated from two or more molecules (section I.B.1), by detecting a signal upon contacting a binding complex of the target biomolecule and a candidate molecule with a reference molecule (section I.B.2), or by detecting a signal upon contacting a binding complex of the target biomolecule and a reference molecule with a candidate molecule (section I.B.3).

### III. Methods for Determining Structure-Activity Relationship and/or Cataloging a Biochemical Entity

Provided herein are methods for determining structure-activity relationship and/or cataloging a candidate biochemical entity or a scaffold thereof. The structure-activity relationship can be, for example, determined using a signature (e.g. SHG, SFG, DFG) of the candidate biochemical entity. In some cases, the signature of a candidate biochemical entity can be compared to the detected signature of another biochemical entity. Similarity in the detected signature can be used to correlate pharmaceutical properties or functional effects.

The signature can, for example, comprise a magnitude, a direction, or a signal per unit time. The signature can also comprise a change in magnitude or direction compared to a baseline, or a change in kinetics. The baseline can be measured prior to contacting the candidate biochemical entity, and optionally after incubation with an agent (e.g. another candidate biochemical entity). Further, the signature can be generated by measuring a kinetic (real time) or an end-point change in the detected signal.

The other biochemical entity can be a known conformational modulator with one or more known pharmacological properties or functional effects (e.g. binding property, functional property, efficacy, potency, selectivity, any combination of the above). In such cases, if the candidate biochemical entity produces a signature similar to that of the known conformational modulator, the candidate biochemical entity can be determined to also have one or more known pharmacological properties or functional effects. Further, if the candidate biochemical entity has one or more common structural parameters [e.g. scaffold, scaffold core, molecular weight, charge, chemical formula, number of total, number of specific atoms (e.g. carbon, hydrogen, nitrogen, oxygen, sulfur), polarity (e.g. as measured by log D, log P, PSA), chemical similarity as measured by graph theory, Tanimoto (or Jaccard) coefficient T, number of aromatic groups, number of heterocyclic groups, number of monocyclic groups, number of fused ring systems, and number of double bonds] with the known conformational modulator, the one or more structural parameters can also be determined to contribute to the one or more known pharmacological properties or functional effects.

Alternatively, the other biochemical entity can be another candidate biochemical entity. If the candidate biochemical entities generate similar signatures (e.g. SHG signatures), the signatures can be used to correlate the candidate biochemical entities to a common pharmacological property or functional effect. This may be applied to an entire library of candidate biochemical entities, wherein the candidate biochemical entities can be grouped or "cataloged" based on the signature. In some examples, the candidate biochemical entities that have a similar signature can further be determined to have a common pharmacological property or functional effect.

### IV. Additional applications

The SHG technique provides a real-time means to detect conformational change with X-ray level sensitivity in solution, and thus allows the target to sample the full ensemble of conformations available to it. The SHG technique may have a major impact on drug discovery. Structural information is highly informative and validating in drug discovery and typically follows biochemical or cell-based assays. The latter assays often generate a number of hits which, upon more stringent examination by a structural technique, turn out to be false positives. It is time-consuming and often impractical to employ NMR or X-ray crystallography to identify (e.g., pick out) real hits (e.g., true positives) (Keseru GM & Makara GM, Drug Discovery Today 11(15-16):741-748, 2006). Our approach combines the features of a biochemical screen, i.e., a molecular-based assay, with a dynamic, structural read-out. In effect, SHG provides a new technique to study structural dynamics and conformational change, which are synonymous with biological function. In the first demonstration of the technique, we identified a single ligand capable of blocking spermine's effect on alpha-synuclein and confirmed by NMR that it appears to function like spermine itself; this in itself is a surprising and unusual result, as there is often a wide gap between the compounds that a primary screen indicates are hits and their subsequent confirmation by a structural technique. Moreover, we identified this ligand against a target for which there are few known binders and no others to the C-terminal region.

SHG screening may be used in variety of modes. For example, one can screen for ligands that inhibit a known ligand-induced conformational change (e.g., antagonist) or which induce a conformational change on their own (e.g., agonist) at one or multiple sites of interest. Types of ligands sought can include lead compounds for therapeutics, allosteric modulators, and tool compounds for chemical biology and disease pathogenesis studies, among others. If a particular assay involves known tool compounds, SHG can be used to provide information about its mechanism of action as well as its binding affinity. Although a significant fraction of most screening libraries contain compounds that are fluorescent and can interfere with the read-out in fluorescence assays, very few are likely SH-active, a more stringent set of physico-chemical properties.

Detecting conformational change in real time in solution is likely to reveal new binding sites, such as allosteric ones, not seen in X-ray experiments. The technique holds great promise as a means to identify allosteric modulators for targets currently considered 'undruggable' such as Ras. With the structural sensitivity of the technique, even weak binders that produce changes in the right direction in conformational space, can be rapidly identified and optimized. In this way, the technique offers a route to identify new classes of drugs that cannot be identified in any other way and to develop drugs with precisely tailored conformational (and thus functional) effects.

Protein conformational change is the primary functional event in most biological processes. Methods to study it generally rely on detecting a change in the signal caused by a change in a molecule's environment rather than a change in its real space coordinates. SHG is highly and directly sensitive to structural changes in real space. As described here, SHG can be used to screen a protein's structural changes site-specifically as they occur in real time, and under physiological conditions so that the protein can sample the full conformational space available to it. These properties make the technique ideal for identifying ligands that modulate a protein's conformation, such as allosteric drugs or endogenous biological modulators. By studying conformational change at multiple probe sites, the technique can provide a global dissection of conformational change and a selective means of identifying and classifying ligands that cause structural changes at those particular set of sites. In some cases, the SHG screening platform may also quantify the angular change (in degrees) of the probe, so that measurements at different sites may provide information to build an atomic-resolution map of conformational change. In short, the technique will have a significant impact in myriad areas of structural biology and drug discovery.

### V. Signatures

The methods herein may include using a surface-selective technique such as, for example, SHG, SFG or DFG to measure conformational states of biochemical entities (e.g., target biochemical entities). In some examples, a conformational state of a biochemical entity can be measured using the surface selective technique to generate a baseline (e.g., SHG baseline). The conformational state of the biochemical entity can then be measured again using the surface selective technique (e.g., following an event, such as contacting a target biochemical entity with a candidate biochemical entity) to generate a signature (e.g.SHG, SFG or DFG signature). The signature can indicate a change in the conformational state of the biochemical entity. For example, the conformational state of a first biochemical entity may change when it is contacted (e.g., bound) with a second biochemical entity. The second biochemical entity may or may not undergo a change in its conformational state. In some cases, both the first and the second biochemical entities may undergo changes in conformation (e.g., in a binding complex between the two). In some cases, the second biochemical entity may undergo a change in conformation that affects the conformation of the first biochemical entity.

In some examples, the baseline may not be measured. For example, a first conformational state of a first biochemical entity can be measured upon contacting the first biochemical entity with a second biochemical entity. Next, a second conformational state of the first biochemical entity can be measured upon contacting the first biochemical entity (e.g., the same copy of the first biochemical entity, or a different copy of the first biochemical entity) with a third biochemical entity, and a difference in the conformational states may be observed by subtracting or comparing the signatures. Thus, in some cases, the signature may be defined with respect to the baseline (e.g., as an SHG, SFG or DFG response with respect to a baseline response). In some cases, the signature may not be defined with respect to a baseline, or may be defined by subtracting a common baseline.

Signatures generated using the surface-selective techniques of the disclosure can comprise nonlinear optical responses (e.g., SHG responses) measured at various times. For example, the signatures can be generated by measuring a kinetic (real time) change in conformational state of the biochemical entity, an end-point change in conformational state of the biochemical entity, or a change in conformational state of the biochemical entity at any other time (e.g., before, during and/or anytime after contacting the biochemical entity with another biochemical entity) or at multiple predetermined time (e.g., before contacting, upon contacting, at 1, 2, 3, 4, 5, 6, 7, 8, 9, 10 or more times after the contacting, or any combination thereof). In some cases, the signature can be generated by measuring nonlinear optical signal intensity (e.g., SHG intensity).

The signatures of the disclosure may be used as basis for screening, selecting and/or evaluating biochemical entities. For example, a biochemical entity can be selected from a group of candidate biochemical entities based on its signature (e.g., the signature of an interaction, such as, for example, binding of a first biochemical entity with a second biochemical entity). In some cases, the selection can include comparing the signature to one or more other signatures (e.g., reference signature of a known compound, signatures of similar biochemical entities, etc.). In one example, the biochemical entity with a change in conformational state that is most similar to a change in conformational state induced by a known conformational modulator is selected. The change in conformational state produced by the known conformational modulator may correlate to a pharmacological property or a functional effect (e.g., mechanism of action, phenotype, clinical property, binding property, etc.). Thus, the comparison may in some cases be used to select biochemical entities with a similar or substantially the same pharmacological property or functional effect. In another example, the signatures of biochemical entities are compared (e.g., the signature of a first biochemical entity is compared to the signature of a second biochemical entity), and a biochemical entity (e.g., in set or library of biochemical entities) is selected based on the comparison. In another example, the biochemical entity is selected based on a correlation of the signature to a known pharmacological property or a known functional effect.

Biochemical entities may have substantially similar or different signatures (e.g., to each other or to reference biochemical entities such as, for example, a known conformation modulator or an approved drug). For example, upon contacting each of the candidate biochemical entities in a screening assay with the same target biochemical entity, each of the candidate biochemical entities may generate a distinct signature.

In some cases, the signatures herein may be correlated to a known pharmacological property or a known functional effect. For example, candidate biochemical entities (e.g., at least two of the candidate biochemical entities) in a screening assay may have a similar pharmacological property or functional effect. The pharmacological property or functional effect (e.g., binding affinity, binding specificity, clinical efficacy, change in phenotype, etc.) may be, for example, at least about 1%, 5%, 10%, 20%, 30%, 40%, 50%, 60%, 70%, 80%, 90%, 95%, or 99% similar, and the like. In another example, candidate biochemical entities (e.g., at least two of the candidate biochemical entities) in a screening assay may not have a similar pharmacological property or functional effect. The pharmacological property or functional effect (e.g., binding affinity, binding specificity, clinical efficacy, change in phenotype, etc.) may be, for example, at most about 1%, 5%, 10%, 20%, 30%, 40%, 50%, 60%, 70%, 80%, 90%, 95%, or 99% similar, and the like. In some cases, a similarity in one or more pharmacological properties or functional effects may correlate with a similarity in corresponding signatures. In other cases, similarity in one or more pharmacological properties or functional effects may not correlate with a similarity in corresponding signatures.

Generating the signatures of the disclosure may comprise measuring one or more nonlinear optical (e.g., SHG) parameters. For example, these parameters may include a change in magnitude of the nonlinear optical (e.g., SHG) signal relative to the nonlinear optical (e.g., SHG) baseline, a change in direction of the nonlinear optical (e.g., SHG) signal relative to the nonlinear optical (e.g., SHG) baseline, a change in rate of the nonlinear optical (e.g., SHG) signal per unit time upon the contacting, or any combination thereof. The signatures may be analyzed using a computer-executable program. In some cases, the signatures may be analyzed for similarity. In some cases, similar signatures may be used in screening methods of the disclosure as basis for selecting (or not selecting) biochemical entities. In some cases, similar signatures may be used in evaluation methods of the disclosure (e.g., generating a structure-activity relationship (SAR) of a set of biochemical entities) as basis for correlating or analyzing the SAR.

In some cases, similarity may be defined using the nonlinear optical (e.g., SHG) parameters. For example, the signature generated by a first biochemical entity and the signature generated by a second biochemical entity may be similar, as determined using one or more nonlinear optical (e.g., SHG) similarity parameters (e.g., any combination of the similarity parameters below). In some examples, the similarity parameter may be that the change in magnitude of a nonlinear optical (e.g., SHG) signal relative to the nonlinear optical (e.g., SHG) baseline upon contacting the first candidate biochemical entity with a target biochemical entity is within less than about 1%, 5%, 10%, 15%, 20%, 25%, 30%, 35%, 40%, 45%, 50%, 55%, 60%, 65%, 70%, 75%, 80%, 90%, or more (e.g., within about 50%) of the change in magnitude of a nonlinear optical (e.g., SHG) signal relative to the nonlinear optical (e.g., SHG) baseline upon contacting the second candidate biochemical entity with the target biochemical entity. In some examples, the similarity parameter may be that the change in direction of a nonlinear optical (e.g., SHG) signal relative to the nonlinear optical (e.g., SHG) baseline upon contacting the first candidate biochemical entity with a target biochemical entity is the same as the change in direction of a nonlinear optical (e.g., SHG) signal relative to the nonlinear optical (e.g., SHG) baseline upon contacting the second candidate biochemical entity with the target biochemical entity. In some examples, the similarity parameter may be that the change in rate of a nonlinear optical (e.g., SHG) signal per unit time upon contacting the first candidate biochemical entity with a target biochemical entity is within a factor of less than about 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.5, 4, 4.5, 5, 6, 7, 8, 9, 10, 15, 20, or more (e.g., about two-fold) of the change in rate of a nonlinear optical (e.g., SHG) signal per unit time upon contacting the second candidate biochemical entity with the target biochemical entity, or vice versa.

In some examples, signatures may include characteristic shapes or profiles of the nonlinear optical (e.g., SHG) signal responses. For example, signatures may have a given number of features, such as, for example, local maxima, local minima, inflection points, overall slope, slope changes, etc. In some cases, these characteristics or features may be measured relative to the nonlinear optical (e.g., SHG) baseline. In some cases, the similarity parameter may be that a nonlinear optical (e.g., SHG) signal measured upon contacting the first candidate biochemical entity with a target biochemical entity and a nonlinear optical (e.g., SHG) signal measured upon contacting the second candidate biochemical entity with the target biochemical entity share at least 1, 2, 3, 4, 5, 6, 7, 8, 9, 10, 15, 20, 30, 40, 50, or more such features (e.g.,. local maxima, local minima, inflection points, overall slope and/or slope changes). In some cases, the similarity parameter may be that one or more of the number of local maxima, local minima, inflection points, overall slope, slope changes and/or other signal features of a nonlinear optical (e.g., SHG) signal measured upon contacting the first candidate biochemical entity with a target biochemical entity is the same as the number of local maxima, local minima, inflection points, overall slope, slope changes and/or other signal features of a nonlinear optical measured upon contacting the second candidate biochemical entity with the target biochemical entity.

### VI. Target biomolecules and Reference Biochemical Entities

The target biomolecule for use in any of the methods described herein can be any biomolecules, e.g., a protein, a DNA, a RNA, or a polysaccharide. The target biomolecule can be a naturally occurring substance, or a subunit or domain thereof, from any natural source, including a virus, a microorganism (including bacterial, fungi, algae, and protozoa), an invertebrate (including insects and worms), the normal or pathological cells of an animal, a vertebrate (such as, a mammal, bird or fish and, among mammals, for example, humans, apes, monkeys, cows, pigs, goats, llamas, sheep, rats, mice, rabbits, guinea pigs, cats and dogs), or the normal or pathological cells of a plant. Alternatively, the target biomolecule can be a non-naturally occurring molecule that has been created *in vitro* or modified such as by a mutation, a chimeric protein, or an artificial protein.

For example, a target protein can be a glyco-, lipo-, phospho-, or metalloprotein. It may be a nuclear, cytoplasmic, membrane-associated, or a secreted protein. The target protein does not need to be a single macromolecule. For example, the target protein may be a homo or hetero-multimer (such as, but not limited to, a dimer, a trimer, or a tetramer) of macromolecules. Additionally, the target protein may require one or more ligands for carrying out physiological functions, such as other proteins, oligo-or polypeptides, nucleic acids, carbohydrates, lipids, or small organic or inorganic molecules or ions. Additional examples include cofactors, ribosomes, polysomes, and chromatin.

Target proteins include but are not limited to intrinsically disordered proteins (IDPs) such as, for example, alpha synuclein (which is implied in Parkinson's) and COW (which is implied in Alzheimer's). Further examples of target proteins include but are not limited to nuclear receptors, orphan nuclear receptor, tyrosine kinase receptors, endothelin, erythropoietin receptor, FAS ligand receptor, protein kinases (e.g., protein kinase C, tyrosine kinases, serine kinases, threonine kinases, nucleotide kinases, or polynucleotide kinases), protein phosphatases (serine/threonine phosphatases, tyrosine phosphatases, nucleotide phosphatases, acid phosphatases, alkaline phosphatases, or pyrophosphatases), cell cycle regulators (cyclin cdk2, CDC2, CDC25, P53, RB), GTPases, Rac, Rho, Rab, Ras, endoproteases, exoproteases, metalloproteases, serine proteases, cysteine proteases, nucleases, polymerases, reverse transcriptases, integrases, ion channels, chaperonins (e.g., heat shock proteins), deaminases, nucleases (e.g., deoxyribonuclease, ribonucleases, endonucleases, exonucleases), telomerases, primases, helicases, dehydrogenases, transferases (peptidyl transferase, transaminase, glycosyltransferases, ribosyltransferases, acetyl transferases, guanylyltransferases, or methyltransferases), hydrolases, carboxylases, isomerases, glycosidases, deaminases, lipases, esterases, sulfatases, cellulases, lyases, reductases ligases or processing enzymes of the cellular ubiquitination pathway (such as E1, E2, or E3 enzymes or deubiquitinases). In some examples, the target proteins for use in any of the methods disclosed herein may be structural and non- structural proteins selected among viral proteins, bacterial proteins, vegetal proteins, animal proteins and human proteins. In some examples, the target protein can be a viral protein, such as, but not limited to, influenza virus, a hepatitis A virus, a hepatitis B virus, a hepatitis C virus, a human immunodeficiency virus, an avian influenza virus, an Ebola virus, a SARS virus, a Hantavirus, or an eastern equine encephalitis virus.

The target protein can be a receptor, including both surface and intracellular receptors. In some examples, the target protein is a nuclear receptor. Nuclear receptors are a family of ligand-activated transcriptional activators. These receptors are organized into distinct domains for ligand binding, dimerization, transactivation, and DNA binding. The steroid receptor family is a large family composed of receptors for glucocorticoids, mineralocorticoids, androgens, progestins, and estrogens. Receptor activation occurs upon ligand binding, which induces conformational changes allowing receptor dimerization and binding of co-activating proteins. These co-activators, in turn, facilitate the binding of the receptors to DNA and subsequent transcriptional activation of target genes. In addition to the recruitment of co-activating proteins, the binding of ligand is also believed to place the receptor in a conformation that either displaces or prevents the binding of proteins that serve as co-repressors of receptor function. If the ligand is a pharmacological agonist, the new conformation is one which interacts with other components of a biological signal transduction pathway, e.g.; transcription factors, to elicit a biological response in the target tissue. If the ligand is a pharmacological antagonist, the new conformation is one in which the receptor cannot be activated by one or more agonists which otherwise could activate that receptor. A non-exhaustive list of NRs is described in International Patent Application Publication No. 2006/046134, (*see* pages 14 and 15, and **Figure 1**). In some examples, the NRs for use in any of the methods disclosed herein can be selected from among an estrogen receptor, an androgen receptor, a glucocorticoid receptor, a retinoic acid receptor alpha (RARG), a retinoic X receptor (RXR), a peroxisome proliferators-activated receptor (PPARs), a liver X receptor alpha (LXRG) or a progesterone receptor.

The target protein can be a G protein-coupled receptor (also known as seven-transmembrane domain receptors), i.e., any member of a superfamily of receptors that mediates signal transduction by coupling with a G protein. GPCRs comprise a large family of transmembrane receptor proteins (representing about 5% of the total genome of humans) that bind to molecules present in the extracellular environment and are capable of triggering signal transduction cascades within the cell and, ultimately, cellular responses. GPCRs are found only in eukaryotes, including yeast, choanoflagellates, and animals. The molecules that bind and activate these receptors include, but are not limited to, light-sensitive compounds, odors, pheromones, hormones, and neurotransmitters, and vary in size from small molecules to peptides to large proteins. One example of a class of GPCR which influences cytosolic calcium levels works through the Gq type of G proteins, which activate a phospholipase C (PLC) pathway, resulting in the hydrolysis of phosphoinositides to generate two classes of different second messengers, namely, diacylglycerol and inositol phosphates. Diacylglycerol, in turn, activates certain protein kinase Cs (PKCs) while inositol phosphates (such as, but not limited to, IP3) stimulate the mobilization of calcium from intracellular stores such as the endoplasmic reticulum, the sarcoplasmic reticulum (for muscle cells), and/or the mitochondria. The molecules that bind and activate these receptors include, but are not limited to, light-sensitive compounds, odors, pheromones, hormones, and neurotransmitters, and vary in size from small molecules to peptides to large proteins.

GPCRs for use in the methods disclosed herein include, but are not limited to, G_{q} protein or G_{q/11}, alpha- 1 adrenegic receptors (α1-AR), urotensin (UT) receptors, 5-HT2 and 5-HT6 serotonin receptors, hypocretic (orexin) receptors, histamine HI receptors, bradykinin B1 and B2 receptors, bombesin BB2 receptors, P2Y purinergic receptors, acetycholine receptors (e.g., M1, M3 and M5), mGluR5 glutamate receptors, vasopressin V2 and VI receptors, angiotensin AGTR1 receptors, cholecystokinin CCKAR and CCKBR receptors, endothelin ENDRA receptors, ghrelin GHSRla receptors, melatonin MTNR1A receptors, neurotensin NTSR1 receptors, platelet-activating factor PTAFR receptors, luteinizing hormone receptors (LHRs), follicle stimulating hormone receptors (FSHRs), gonadotrophic releasing hormone receptors (GnRHRs), and prolactin releasing peptide receptor PRLHR receptors. In some examples, the GPCR is endogenously expressed in the cell expressing the calcium sensor protein. In other examples, the GPCR is heterologously expressed in the cell expressing the calcium sensor protein.

The target protein can be a kinase. A kinase is a type of enzyme that transfers phosphate groups from high-energy donor molecules, such as ATP, to specific substrates, a process referred to as phosphorylation. One of the largest groups of kinases is protein kinases, which act on and modify the activity of specific proteins. Kinases are used extensively to transmit signals and control complex processes in cells. More than five hundred different kinases have been identified in humans. Their enormous diversity, as well as their role in signaling, makes them an object of study particularly with regard to disease states characterized by aberrant kinase expression or regulation.

Protein kinases contain a large flexible loop, called the activation loop or A-loop, whose conformation is believed to regulate kinase activity. In many kinases, the conformation of the A-loop is controlled by the phosphorylation of specific residues within this region (Johnson 1996). The activation loop generally begins with a conserved AspPheGly sequence and ends at a conserved AlaProGlu. In structures of inactive kinases, this loop often blocks either the substrate or ATP binding sites (Hubbard 1994; Mohammadi 1996; and McTigue 1999). Tyrosine kinases usually have one or two tyrosines in the loop, MAPK kinases have a T[DE]Y motif, which is phosphorylated on both T and Y, while most other kinases have a threonine within the loop.

The target proteins for use in the methods of the disclosure are broadly applicable to any protein kinase. These can include protein tyrosine kinases and protein serine kinases. Non-limiting examples of protein tyrosine kinases are pp60c-src, p561ck, ZAP kinase, platelet derived growth factor receptor tyrosine kinase, Bcr-Abl, VEGF (vascular endothelial growth factor) receptor tyrosine kinase, and epidermal growth factor receptor tyrosine kinase, and epidermal growth factor receptor-like tyrosine kinases. Non-limiting examples of serine protein kinases applicable for use in the present disclosure include MAP (mitogen activated protein) kinase, protein kinase C, protein kinase A, Akt, and CDK (cyclin dependent protein kinase). In mammalian biology, protein kinases belonging to the mitogen activated protein kinase (MAPK) family are inappropriately activated in a variety of proliferative cell diseases (such as, for example, cancers) associated with the mutation of ras genes and deregulation of growth factor receptors (Magnuson et al., Seminars in Cancer Biology, 5:247-252 (1994)). MAP kinases are known in the art and a partial non-limiting list of such kinases includes abl, Aurora-A, Aurora-B, Aurora-C, ATK, bcr-abl, Blk, Brk, Btk, c-Kit, c-Met, c-Src, CDK1, CDK2, CDK4, CDK6, cRafl, CSF1R, CSK, EGFR, ErbB2, ErbB3, ErbB4, ERK, Fak, fes, FGFR1, FGFR2, FGFR3, FGFR4, FGFR5, Fgr, FLK-4, Flt-1, Fms, Fps, Frk, Fyn, Hck, IGF-1R, INS-R, Jak, KDR, Lck, Lyn, MEK, p38, PDGFR, PIK, PKC, PYK2, Ros, Tiel, Tie2, Trk, Yes and Zap70. In some examples of the methods described herein, the kinase is abl kinase.

With respect to kinases, several types of compounds are known to modulate the function of kinases. For example, type I kinase inhibitors recognize the active conformation of a kinase. They bind to the ATP-binding site by presenting one to three hydrogen bonds which mimic the hydrogen bonds normally formed by ATP. Without being bound to theory, it is believed that, in contrast to type I kinase inhibitors, type II kinase inhibitors recognize the inactive conformation of a kinase and can indirectly compete with ATP by occupying the hydrophobic pocket directly adjacent to the ATP-binding site. This hydrophobic pocket is created by the unique DFG-out conformation of the activation loop. While this is not necessary for functionality, some type II inhibitors are able to form a hydrogen bond directly to the ATP-binding site (Gotink & Verheul, Angiogenesis, 2010, 13(1): 1-14). Type III kinase inhibitors, on the other hand, are non-ATP competitive kinase inhibitors which modulate kinase activity by binding to sites other than the activation loop (i.e., by binding to allosteric sites on the kinase). Due to the fact that Type III compounds bind to less-conserved sites on kinases, they are highly selective and are of increasing interest to the research and drug discovery communities.

The Ras family of small GTPases is composed of intracellular signaling molecules which are regulated by the nucleotide guanosine-5'-triphosphate (GTP). These proteins function as binary signaling switches with both "on" and "off" states. In the "off" state, Ras is bound to the nucleotide guanosine diphosphate (GDP), while in the "on" state, Ras is bound to GTP. Hence, activation and deactivation of Ras are controlled by cycling between the active GTP-bound and inactive GDP-bound forms. In the GTP-bound conformation, Ras has high affinity for numerous effectors which allow it to carry out its intracellular functions. Ras-regulated signaling pathways control processes as diverse as actin cytoskeletal integrity, proliferation, differentiation, cell adhesion, apoptosis, and cell migration. In some cases, mutations result in constitutive Ras activation (i.e., a Ras protein with the inability to adopt its "off' state, irrespective of whether it is bound to GDP), leading to aberrant signaling. The methods herein can be used to screen or identify compounds capable of specifically modulating the behavior of Ras (e.g., in cancer cells) to decrease the aberrant signaling of downstream Ras effector molecules.
Any Ras protein is contemplated for use in any of the methods disclosed herein. The three human ras genes encode extremely homologous proteins made up of chains of 188 to 189 amino acids, designated H-RAS, N-RAS and K-RAS4A and K- RAS 4B (the two K- RAS proteins arise from alternative splicing). While the clinically most notable members of the Ras subfamily are H-RAS, K-RAS and N-RAS, predominantly for being implicated in many types of cancer, there are many other members of this subfamily as well which can include, but are not limited to, DIRAS1, DIRAS2, DIRAS3, ERAS, GEM, MRAS, NKIRAS1, NKIRAS2, NRAS, RALA, RALB, RAP1A, RAP1B, RAP2A, RAP2B, RAP2C, RASD1, RASD2, RASL10A, RASL10B, RASL11A, RASL11B, RASL12, REM1, REM2, RERG, RERGL, RRAD, RRAS, or RRAS2 (*see also,* Wennerberg et al., 2005, "The Ras superfamily at a glance," J. Cell. Sci. 118 (Pt 5): 843-6,). Also contemplated for use within the scope of the methods of the disclosure are mutant forms of the Ras protein.

The target protein includes a mutant form of target protein. As used herein, a "mutation" includes an amino acid residue deletion, an amino acid residue insertion, and/or an amino acid residue substitution of at least one amino acid residue in a defined primary amino acid sequence, such as a primary amino acid sequence of a target protein. An amino acid "substitution" means that at least one amino acid component of a defined primary amino acid sequence is replaced with another amino acid (for example, a cysteine residue or a lysine residue). Methods for engineering a mutation or substitution into the primary amino acid sequence of a target protein are well known in the art via standard techniques. The target proteins for use in the methods described herein may include conservative substitutions.

Substantial modifications in the biological properties of target proteins are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Non-conservative substitutions will entail exchanging a member of one of these classes for another class.

In further examples, mutant target proteins for use in any of the methods disclosed herein may comprise one or more non-naturally occurring or modified amino acids. Non-natural amino acids include, but are not limited to homo-lysine, homo-arginine, homo-serine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, citrulline, pentylglycine, pipecolic acid and thioproline. Modified amino acids include natural and non-natural amino acids which are chemically blocked, reversibly or irreversibly, or modified on their N-terminal amino group or their side chain groups, as for example, N-methylated D and L amino acids, side chain functional groups that are chemically modified to another functional group. For example, modified amino acids include methionine sulfoxide; methionine sulfone; aspartic acid- (beta-methyl ester), a modified amino acid of aspartic acid; N-ethylglycine, a modified amino acid of glycine; or alanine carboxamide, a modified amino acid of alanine. Additional non-natural and modified amino acids, and methods of incorporating them into proteins and peptides, are known in the art (see, e.g., Sandberg et al., (1998) J. Med. Chem. 41: 2481-91; Xie and Schultz (2005) Curr. Opin. Chem. Biol. 9: 548-554; Hodgson and Sanderson (2004) Chem. Soc. Rev. 33: 422-430).

In some examples, mutant target proteins for use in the methods described herein can be isolated from cells (such as a cancer cell) by an appropriate purification scheme using standard protein purification techniques. In another example, mutant target proteins for use in the instantly described methods are produced by recombinant DNA techniques. Alternative to recombinant expression, mutant target proteins for use in the methods described herein can be synthesized chemically using standard peptide synthesis techniques.

Further, reference biochemical entities can be subject to the methods described in the present application to generate reference signatures, which can be used to correlate the signatures of candidate biochemical entities to pharmacological properties and/or functional effects. For example, a reference biochemical entity with one or more known pharmacological properties and/or functional effects can have a reference signature that can be compared to the signatures of a library of candidate biochemical entities. A candidate biochemical entity that has a similar signature to reference signature can be correlated to and/or further tested for the one or more known pharmacological properties and/or functional effects.

The reference biochemical entity can be any known drug with a known function and/or a known target. The reference biochemical entity can be, for example, an anticancer drug or an anti-inflammation drug. The reference biochemical entity can also be a known kinase inhibitor including but not limited to tyrosine kinase inhibitiors and serine/threonine kinase inhibitors. Examples of reference biochemical entities include but are not limited to Abilify, Nexium, Humira, Crestor, Advair Diskus, Enbrel, Cymbalta, Remicade, Neulasta, Copaxone, Lantus Solostar, Rituxan, Spiriva, Januvia, Atripla, Lantus, Avastin, Lyrica, OxyContin, Epogen, Celebrex, Truvada, Diovan, Gleevec, Herceptin, Lucentis, Vyvanse, Zetia, Namenda, Levemir, Symbicort, Tecfidera, AndroGel, Novolog, enoxaparin, methylphenidate, Suboxone, Xarelto, ProAir HFA, Humalog, Alimta, Victoza, Viagra, Seroquel XR, Synagis, Avonex, Renvela, Rebif, Cialis, Gilenya, Nasonex, Stelara, Restasis, budesonide, acetaminophen/hydrocodone, Flovent HFA, Lovaza, Prezista, Isentress, Janumet, Procrit, doxycycline, Orencia, amphetamine/dextroamphetamine, VESIcare, Dexilant, Neupogen, lidocaine, Lunesta, fenofibrate, Zytiga, Reyataz, Sensipar, metoprolol, Invega Sustenna, Synthroid, Avonex, Prevnar 13, Xolair, levothyroxine, Benicar, Stribild, Zostavax, Pradaxa, Vytorin, Tamiflu, Xgeva, Evista, Xeloda, Aranesp, Ventolin HFA, divalproex sodium, Afinitor, Betaseron, Adderall XR, and Complera.

### VII. Nonlinear-active moieties

In some examples, one or more nonlinear-active (e.g., second harmonic-active) moieties or labels can be attached to or incorporated into a target biochemical entity (e.g., biomolecule), a candidate biochemical entity (e.g., candidate molecule), a reference biochemical entity (e.g., molecule), or any combination thereof. In some examples, such as, for example, in assays and methods for screening structurally similar biochemical entities or evaluating biochemical entities by generating a structure-activity relationship, the nonlinear-active moieties or labels can be attached to or incorporated into the target biochemical entity, one or more candidate biochemical entities in a library of structurally similar biochemical entities, one or more biochemical entities in a set of biochemical entities with distinct molecular structures, the reference biochemical entity, or any combination thereof. For example, in any given assay where a first biochemical entity is contacted (e.g., resulting in a mutual interaction, such as, for example, a binding event) with a second biochemical entity, the first biochemical entity and/or the second biochemical may comprise a label suitable for measuring conformational change using the surface-selective technique described herein (e.g., SHG, SFG or DFG).

In some cases, the label can be a second harmonic-active moiety or label (a.k.a., an SHG-moiety or SHG-label). In some cases, a sum frequency-active or difference frequency-active moiety or label. In some cases, the nonlinear-active moieties or labels (also "moieties" or "labels" herein) may be SHG-active, SFG-active, DFG-active, or a combination thereof.

In some examples, the target biochemical entity (e.g., a biomolecule such as a protein, a cell, etc.) can be labeled in one or more predetermined locations. The label(s) may be placed, for example, at a given distance from (e.g., at or adjacent to) an active site, a binding site, an allosteric binding site, or any other sites on or within the biochemical entity (e.g., protein) structure (e.g., any site that may undergo conformational change screened by the assay, any site undergoing conformational change correlating to an *in vivo* or *in vitro* pharmacological property, etc.).

In some cases, such as, for example, to enable the screening and evaluation assays of the disclosure, multiple copies of the same target biochemical entity may be labeled in the same location(s) on or within the target biochemical entity. For example, the systems and methods of the present disclosure can be used to detect differences in changes in conformational state of the target biochemical entity contacted with biochemical entities having very similar structures. Further, the differences in changes in conformational state may correlate with a pharmacological property or a functional effect. Thus, identical label placement may ensure that the biochemical entities interacting with the target biochemical entity are evaluated under the same conditions, and that observed differences in changes in conformational state of the target biochemical entity are due to differences in at least one of structure, pharmacological property, functional effect, etc., rather that different label placement.

Each label may have a given location (e.g., 3D coordinate) on the target biochemical entity. A given label may have a first location (e.g., 3D coordinate (x,y)i) on or within a first copy of the target biochemical entity and a second location (e.g., 3D coordinate (x,y)₂) on or within a second copy of the target biochemical entity. In some examples, a relative difference between the first location and the second location (e.g., |(x,y)₁-(x,y)₂|/(x,y)₁ or |(x,y)₁-(x,y)₂|/(x,y)₂) may be less than about 75%, 50%, 25%, 10%, 5%, 1%, and like. In some examples, an absolute difference between the first location and the second location may be less than about 1%, 2%, 3%, 4%, 5%, 6%, 7%, 8%, 9%, 10%, 15%, 25%, 50%, or 75% of a diameter, height, width, breadth or other characteristic dimension of the target biochemical entity.

The moieties (such as, a label) can be bound, either covalently or non-covalently, to a target biomolecule in order to render the resulting target biomolecule susceptible to second harmonic generation and amenable to study at an interface using a surface-selective technique. The labeled target biomolecules may then be studied by surface-selective techniques such as second harmonic generation or sum-frequency generation. The exogenous moieties (such as, a label) can be pre-attached to the target biomolecule, and any unbound or unreacted labels separated from the labeled entities before a measurement is made. In one example, the second harmonic-active moiety (such as, a label) is attached to the target biomolecule *in vitro.*

In some cases, at least two distinguishable second harmonic-active moieties (such as, labels) can be used. The orientation of the attached two or more distinguishable labels would then be chosen to facilitate well defined directions of the emanating coherent nonlinear light beam. The two or more distinguishable labels can be used in assays where multiple fundamental light beams at one or more frequencies, incident with one or more polarization directions relative to the sample, are used, with the resulting emanation of at least two nonlinear light beams. In one example, the second harmonic-active moiety (such as, a label) comprises a plurality of individual second harmonic-active moieties (such as, labels) which each have a nonlinear susceptibility and are bound together in a fixed and determinate orientation with respect to each other so as to increase the overall nonlinear susceptibility of the second harmonic-active moiety (such as, a label).

In some cases, the second harmonic-active moiety (such as, a label) is a dye. The target biomolecule can be labeled by a dye through specific labeling or non-specific labeling.

The second harmonic-active moiety can be attached to a target biomolecule through specific labeling, e.g., via a covalent bond or a hydrogen bond. For example, the second harmonic-active moiety (such as, a label) can be covalently or non-covalently attached to an amine group, a lysine group, or a sulfhydryl group in the primary amino acid sequence of the target protein to be detected. In some examples, the second harmonic-active moiety (such as, a label) possesses an amine-reactive succinimidyl ester, a thiol-reactive maleimide, or an aldehyde- and/or ketone-reactive hydrazide and hydroxylamine. In some examples, the SFG or SHG-active dye label can be conjugated via "click chemistry" for coupling to azides. Details of click chemistry for use in conjugate formation are described in: "Synthesis and Functionalization of Biomolecules via Click Chemistry, C. Schilling et al, Chapter 15 pages 355-378 in *"*Click Chemistry for Biotechnology and Materials Science" J. Lahann (Ed), Wiley (2009).

Examples of dyes appropriate for use as second harmonic or sum frequency-active moieties (such as, a label) in the methods disclosed herein include maleimide labels (such as PyMPO-MALEIMIDE™ (1-(2-Maleimidylethyl)-4-(5-(4-Methoxyphenyl)Oxazol-2-yl)Pyridinium Methanesulfonate), which specifically labels proteins on thiol containing residues, typically cysteines), PyMPO-NHS (which specifically labels primary amine containing residues, typically lysinzes), oxazole labels (such as PyMPO-succinimidyl ester which specifically labels amines), BADAN™ (6-Bromoacetyl-2-Dimethylaminonaphthalene), and ACRYLODAN™ (6-Acryloyl-2-Dimethylaminonaphthalene). In other examples, the labels can be coumarin-based dyes such as, but not limited to, ketocoumarin, and 3,3'-carbonyl bis (7-diethylaminocoumarin). In other examples, the label can be PyMPO-SE™ (1-(3-(Succinimidyloxycarbonyl)Benzyl)-4-(5-(4-Methoxyphenyl)Oxazol-2-yl)Pyridinium Bromide).

In some cases, a native amino acid residue in the primary amino acid sequence of the target protein can be mutated or substituted with another amino acid that is capable of binding to a second harmonic-active dye. The target proteins described herein may include conservative substitutions.

In other cases, the second harmonic-active moiety (such as, a label) may be an unnatural amino acid (UAA). In contrast to conventional labels, UAA's offer a means of labeling proteins at both buried and exposed sites. Additionally, as innate components of the protein, they can report structural changes with more sensitivity and fidelity than labels (such as dyes) attached to amino acid functional groups (e.g., cysteines, amines, etc.). UAAs's possess hyperpolarizability for detecting proteins using a nonlinear technique such as second harmonic generation. Therefore, these specific unnatural amino acids have also been referred to as SHAA's ("Second harmonic Amino-Acid"). Another advantage of using UAA's as probes for detection of changes in protein structural confirmation is that the detection can be carried out *in vivo-*that is, in live cells. For example, the methods described herein can be used to detect the signal exhibited by a target protein in live cells in response to binding of a drug candidate.

Any hyperpolarizable UAA can be used as a second harmonic-active moiety (such as, a label). Examples of UAAs include Aladan (Cohen et al., 2002, Science, 296:1700; Abbyad et al., 2007, J. Phys. Chem., 111:8269, dansylalanine (Summerer et al., Proc. Nat. Acad. Sci. U.S.A., 2006, 103(26): 9785-9789). In one example, the unnatural amino acid is sum-frequency generation-active (SFG-active). In other examples, the UAA is not hyperpolarizable, but possesses the appropriate chemical functional group or groups to permit it to bind to a second harmonic-active label dye, such as any of the dyes described above. In other examples, the UAA can include a probe with tailored vibrational properties for engineering into discreet sites within a protein to identify site-specific conformational changes by SFG. In some examples, probe moieties for inclusion into UAAs desirably are small enough so that they do not perturb native protein structure and can include, but are not limited to, NO, CN, SCN or N₃. In some examples, the probe moieties provide unique vibrational signatures in the spectral range of between about 1,900 and 2,300 cm⁻¹, which is well separated from intrinsic protein vibrations. In another example, a UAA can be used to attach the target protein to a surface, such that a second harmonic-active moiety (such as, a label) possesses a net orientation with respect to the surface.

In some examples, the unnatural amino acids contain isotopic probe moieties such as deuterium, 13C, or other isotopes. An illustrative example of the use of isotopic labeling (such as deuteration) is set forth in Weidner et al., "Sum frequency generation and solid-state NMR study of the structure, orientation, and dynamics of polystyrene-adsorbed peptides," 13288-13293, PNAS, July 27, 2010, vol. 107, no. 30*.* In some examples, isotopic labeling of a native (i.e., naturally occurring) amino acid or amino acid residue in the primary amino acid sequence of the target protein may be used. In some examples, isotopic labeling of natural and unnatural amino acids may be used in concert. Further, is some examples, isotopic labeling of any biochemical entity (candidate, target, or both) may be used. Thus, isotopic labeling may not only include amino acids, but any atoms or moieties in the biochemical entities provided herein. In some cases, isotopic labels or probes may also be attached to biochemical entities herein.

Accordingly, in some cases, structural changes in a target protein can be determined in real time and real space by measuring the tilt angle or absolute tilt angle of an unnatural amino acid label, or a series of such labels, engineered into the amino acid sequence in different mutants of the target protein. The probes can be incorporated at any site within the target protein or at its termini, or in any domain thereof. In some examples, the target protein can include a second harmonic-active label that is chemically equipped to react covalently with a UAA. For example, if the UAA incorporated into a protein is Para-acetyl-phenylalanine (pAcF), the second harmonic-active dye would have appropriate chemistry on it for bonding covalently to pAcF. In another example, the incorporation of a SHAA in addition to a second UAA, the second UAA (which will in general not be second harmonic-active) allows chemically orthogonal covalent coupling of the protein in an oriented manner to a surface derivatized with appropriate chemistry for reaction with the second UAA. With a highly oriented target protein sample that is SH-active (using the two UAA's), both the baseline SHG signal and the contrast (change in signal with conformational change) can be larger in comparison to target proteins which do not utilize UAA's to produce SHG signals.

In other cases, use of one or more UAA's in the amino acid sequence of a target protein in any of the methods disclosed herein enables the determination of the actual conformational change the target protein undergoes upon binding to a candidate allosteric modulator by determining the tilt angle of one or more labels at one or more sites within the target protein as a function of time. The three dimensional structure of the target protein can be determined by making one or more mutants of a protein each containing a SHAA probe placed in a different location (i.e., the probe orientation relative to the surface in each mutant, and therefore the side-chain orientation, can be determined for the probe in each mutant and a model of the overall three dimensional protein structure can be built using this information). Information from steric hindrance methods, statistical methods, molecular dynamics, Ramachandran plots, or energy minimization methods known to those skilled in the art can be used to further aid in determining the structure given the measured probe tilt angles. A time-resolved measurement of the tilt angle of a probe produces a motion picture of a conformational change of a protein as it occurs in real time. Because of SHG's (and SFG's) virtually instantaneous response and sensitivity, spatial orientation of a particular probe (e.g., tilt angle or absolute tilt angle relative to a surface) can be measured in real time at any time scale of interest provided signal strength is sufficient.

Further information related to the use of UAA's in SHG techniques can be found in U.S. Patent Application Publication No. 2010/0068144.

### VIII. Interfaces

In some aspects of the methods disclosed herein, the target protein is bound to a solid surface or oriented with respect to an interface such that a second harmonic-active-label bound to the target protein has a net orientation. It is this net orientation that can change upon binding within the target protein active site, or upon binding of a candidate agent capable of stabilizing the structure of a mutant or wild type target protein into an inactive or active conformation, provided that the agent induces a conformational change in the structure of the labeled target protein. In some examples, the interface can be made of silica, glass, silicon, polystyrene, nylon, plastic, a metal, semiconductor or insulator surface, or any surface to which biological components can adsorb or be attached. In different examples, the interface can be a vapor-liquid interface, a liquid-liquid interface, a liquid-solid, or a solid-solid interface. In one example, the vapor-liquid interface is an air-water interface. In one example, the liquid-liquid interface is an oil-water interface. In different examples, the liquid-solid interface is a water-glass interface or a benzene-SiO₂ interface.

In some aspects, the interface can also include biological cell and liposome surfaces. The attachment or immobilization can occur through a variety of techniques well known in the art. For example, with proteins, the surface can be derivatized with aldehyde silanes for coupling to amines on surfaces of biomolecules (MacBeath and Schreiber, 2000). BSA-NHS (BSA-N-hydroxysuccinimide) surfaces can also be used by first attaching a molecular layer of BSA to the surface and then activating it with N,N'-disuccinimidyl carbonate. The activated lysine, aspartate or glutamate residues on the BSA react with surface amines on the proteins.

Supported phospholipid bilayers can also be used, with or without membrane proteins or other membrane associated components as, for example, in Salafsky et al., Biochemistry, 35, 14773-14781, 1996-relevant portions of which are incorporated by reference herein by reference, *"*Biomembranes", Gennis, Springer-Verlag, Kalb et al., 1992, and Brian et al., 1984. Supported phospholipid bilayers are well known in the art and there are numerous techniques available for their fabrication, with or without associated membrane proteins. These supported bilayers typically must be submerged in aqueous solution to prevent their destruction when they become exposed to air. In some examples, the surface is a lipid analog bilayer surface.

If a solid surface is used (e.g., planar substrate, beads, *etc*.) it can also be derivatized via various chemical reactions to either reduce or enhance its net surface charge density to optimize the detection of target protein-candidate allosteric modulator interactions. In other examples, the solid surface can be a glass surface, a plastic surface, a metal surface, a latex surface, a rubber surface, a ceramic surface, a polymeric surface, a polypropylene surface, a polyvinylidene difluoride surface, a polystyrene surface, or a polyethylene surface (such as a polyethylene glycol surface). The support on which the target proteins are immobilized may be composed from a wide range of material, such as, but not limited to, biological, nonbiological, organic, inorganic, or a combination of any of these, existing as particles, strands, precipitates, gels, sheets, tubing, spheres, containers, capillaries, pads, slices, films, plates, or slides. The surface may have any convenient shape, such as, but not limited to, a disc, square, sphere, or circle. The surface can be preferably flat but may also take on a variety of alternative surface configurations. For example, the surface may contain raised or depressed regions on which a sample (such as a protein) is located. The surface preferably forms a rigid support on which the sample can be formed. The surface is also chosen to provide appropriate light-absorbing characteristics. For example, the surface may be, without limitation, a polymerized Langmuir Blodgett film, functionalized glass, Si, Ge, GaAs, GaP, SiO₂ SiN₄, modified silicon, or any one of a wide variety of gels or polymers such as (poly) tetrafluoroethylene, (poly)vinylidenedifluoride, polyethylene glycol, polystyrene, polycarbonate, or combinations thereof. Other surface materials will be readily apparent to those of skill in the art. In one example the substrate is flat glass or silica.

In some aspects, the surface can be etched using well known techniques to provide for desired surface features. For example, by way of the formation of trenches, v-grooves, mesa structures, or the like, the target proteins (such as, synthesis regions of proteins) may be more closely placed within the focus point of impinging light. The surface may also be provided with reflective "mirror" structures for maximization of emission collected therefrom. As another example, the surface can be etched to form wells.

In another aspect of the present disclosure, oligo-polyethylene glycol (PEG) molecules can be used for immobilizing an affinity-tagged target protein to a surface for SHG or SFG detection. In some examples, the PEG can be SAT(PEG4) (*N*-Succinimidyl S-acetyl(thiotetraethylene glycol). A pegylated interface suitable for detecting SHG signals can be prepared by coating a suitable surface, such as any of the surfaces described above, with an oligo PEG solution. In one example the surface can be glass. In another example, the surface can be amino-terminated silane derivatized glass. Affinity tags are common in the art and may be, for example, a histidine tag (such as a His₆ tag), a maltose binding protein tag, an HA tag, a biotin tag, a thiol tag, or a GST tag. In some examples, the affinity tag is a histidine having any of 1, 2, 3, 4, 5, 6, 7, 8, 9, 10, 11, 12, 13, 14, 15, 16, 17, 18, 19, 20, or more histidine residues. In one example, the oligo-PEG molecules are modified with an agent that will bind to the affinity tag expressed on the target protein. The agent can be nickel or cobalt, in the case of a histidine tag, or it can be a sugar (such as maltose), an antibody, or any other molecule known in the art that is capable of binding to an affinity tag.

### IX. Kits

The present disclosure provides a kit comprising compositions for use in the practice of the methods described herein. For example, a kit can comprise one or more SHG, SFG or DFG-active moieties (such as labels) for conjugation to target biochemical entities (e.g., target proteins), such as any of the SHG, SFG or DFG-active labels (such as dye labels) described herein. These can include dye labels with different coupling chemistries for specific functional groups such as, but not limited to, maleimide labels (such as, PyMPO-MALEIMIDE™ (1-(2-Maleimidylethyl)-4-(5-(4-Methoxyphenyl)Oxazol-2-yl)Pyridinium Methanesulfonate)), PyMPO-NHS, oxazole labels, BADAN™ (6-Bromoacetyl-2-Dimethylaminonaphthalene), PyMPO-SE™ (1-(3-(Succinimidyloxycarbonyl)Benzyl)-4-(5-(4-Methoxyphenyl)Oxazol-2-yl)Pyridinium Bromide), and ACRYLODAN™ (6-Acryloyl-2-Dimethylaminonaphthalene). In other examples, the dyes can be coumarin-based dyes such as, but not limited to, ketocoumarin, and 3,3'-carbonyl bis (7-diethylaminocoumarin).

Also contemplated for inclusion in a kit are tools and reagents for labeling proteins with SHG, SFG or DFG-active labels for use in any of the methods disclosed herein. These can include, without limitation, buffer panels for testing different labeling conditions, buffers for performing a labeling reaction, tubes in which to carry out labeling reactions, spin columns and/or gel filtration columns for purifying labeled protein from unbound label, and buffers for washing the labeled protein.

In another example, the kits can comprise one or more surfaces for binding a target biochemical entity (e.g., protein), such as any of the surfaces disclosed herein. These surfaces can either be reusable or consumable (i.e., intended for one or a limited number of uses). The surfaces can be made of glass (such as a glass slide), plastic, metal, latex, rubber, ceramic, polymer (such as, but not limited to, polypropylene, polyvinylidene difluoride, polystyrene, or polyethylene (for example, polyethylene glycol)) or any surface to which biochemical entities (e.g., biological molecules such as proteins) can adsorb or be attached. In some examples, the surfaces are smooth. However, in other examples, the surfaces can include preformed surface features, such as, but not limited to, trenches, v-grooves, mesa structures, and/or wells. Additionally, the surfaces can be pre-cleaned according to any of the methods disclosed herein. In additional examples, where the surface does not include preformed surface features, the kits can contain adhesive gaskets for templating wells on the surface.

In one example, the surfaces included in the kit can be optimized for different excitation geometries, wherein the mode of delivering or generating nonlinear optical light is based on, without limitation, one or more of TIR (total internal reflection), transmission, or reflection.

In a further example, the surfaces included in the kits disclosed herein can be pre-coated with one or more molecules which provide a surface chemistry suitable for attachment to a target biochemical entity (e.g., target protein). These molecules can include any of those disclosed herein such as, but not limited to, aldehyde silanes, BSA-NHS (BSA-N-hydroxysuccinimide), or oligo-polyethylene glycol (PEG). In one example, the molecule can include an affinity tag, such as any of those disclosed herein. For example, the surface can be coated with nickel-oligo-PEG molecules for immobilizing histidine-tagged proteins to the surface.

In another example, the surfaces included in the kits can be pre-coated with a supported lipid (such as, but not limited to, phospholipid) bilayer or supported lipid analog bilayer. This example of the kits also includes lipids or mixtures of lipids for use in preparing supported lipid bilayers or supported lipid analog bilayers on any of the surfaces disclosed herein. In some examples, the lipid mixture can comprise phosphocholine such as, but not limited to, DOPC (1,2-dioleoyl-*sn*-glycero-3phosphocoline). In some examples, the lipid mixture can comprise, without limitation, one or more of DDAB (N,N-distearyl-N,N-dimethylammonium bromide), DMRIE (N-(1,2-dimyristyloxyprop-3-yl)-N,N-dimethyl-N-hydroxyethyl ammonium bromide), DODAC (N,N-dioleyl-N,N-dimethylammonium chloride), DOGS (diheptadecylamidoglycyl spermidine) DOPE (1,2-*sn-*dioleoylphoshatidyethanolamine), DOSPA (N-(1-(2,3-dioleyloxy)propyl)-N-(2-(sperminecarboxamido)ethyl)-N,N-dimethylammonium trifluoroacetate), DOTAP (N-(1-(2,3-dioleyloxy)propyl)-N,N,N-trimethylammonium chloride), DOTMA N-(1-(2,3-dioleyloxy)propyl)-N,N,N-trimethylammonium chloride). In a further example, any of the lipids or lipid components of lipid mixtures can include an affinity tag for binding to proteins (such as, but not limited to, a nicklated lipid, for example, DOGS-Ni-NTA lipids).

Also contemplated for inclusion in one or more of the kits disclosed herein are signal peripherals for use with an apparatus for detection of interactions between biochemical entities (e.g., target protein-drug candidate interactions) and effects on target biochemical entity (e.g., protein) conformational structure, for determining specificity of a binding interaction between a target biochemical entity (e.g., biomolecule) and another biochemical entity (e.g., a drug candidate). These can include a pass-filter, a color filter, an interference or other spectral filter (for wavelength selection or to separate the fundamental(s) from higher harmonics), one or more polarizing optics, and/or one or more mirrors or lenses for directing and focusing beams. Motorized rotational stages for purposes of making quantitative measurements can also be included in the kits disclosed herein.

In another example, one or more co-factors necessary for protein or enzymatic function can be included in the kits disclosed herein. These can include inorganic co-factors (such as, but not limited to, copper, iron, magnesium, manganese, molybdenum, nickel, cobalt, or zinc ions). These can also include organic cofactors such as, but not limited to, thiamine pyrophosphate, NAD⁺ (NADH), NADP⁺ (NADPH), pyridoxal phosphate, lipoamide, methylcobalamin, cobalamine, biotin, Coenzyme A, tetrahydrofolic acid, menaquinone, ascorbic acid, flavin mononucleotide, flavin adenine dinucleotide, Coenzyme F420, adenosine triphosphate (ATP), adenosine diphosphate (ADP), guanosine triphosphate (GTP), guanosine diphosphate (GDP), S-adenosyl methionine, Coenzyme B, Coenzyme M, Coenzyme Q, cytidine triphosphate, glutathione, heme, nucleotide sugars, pyrroloquinoline, quinone, and/or tetrahydrobiopterin. Also contemplated for inclusion in any of the kits disclosed herein are one or more labeled (such as SFG or SHG-labeled) peptides and/or proteins known to bind to one or more target proteins (for example, ubiquitin or SUMO) or one or more SFG or SHG-labeled antibodies or fragments thereof.

In still other examples, the kits can include one or more components for introducing SHG-, SFG-, or DFG-active unnatural amino acid (UAA) labels into biochemical entities (e.g., proteins). These components can include, without limitation, cell free translation extracts, tRNA/tRNA-synthetase pairs for introducing a particular unnatural amino acid label (such as, Aladan) and/or reaction and wash buffers.

In another example, the kits can include one or more components for live cell labeling. These can include, without limitation, surfaces for cell attachment, SHG-, SFG-, or DFG-active labels, buffer for use in the cell labeling reaction, wash-out buffer for removing unreacted label, as well as cell growth media.

In some examples, these components may allow labeling to be performed selectively in one or more locations on biochemical entities (e.g., the target protein), such as, for example, in strategic locations at a given distance from (e.g., at or adjacent to) an active site, a binding site, an allosteric binding site, or any other sites on or within the protein structure (e.g., any site that may undergo conformational change screened by the assay, any site undergoing conformational change correlating to an *in vivo* or *in vitro* pharmacological property, etc.). The kits may include tools for achieving selective labeling at predetermined positions. For example, the kits may include tools for labeling multiple target biochemical entities in the same location, which may enable signature-based screening and evaluation as described elsewhere herein.

### X. Systems

The present disclosure also provides systems for use in the practice of the methods described herein. In some examples, the system comprises a target protein labeled with an SHG-, SFG- or DFG-active moiety (such as any of the SFG-, SHG- or DFG-active moieties disclosed in any of the compositions, methods, or kits provided herein, for example, an SFG-, SHG-or DFG-active dye label or an SFG-, SHG- or DFG-active unnatural amino acid). The system also comprises an apparatus for detection of target protein- agent interactions comprising i) a source of the fundamental light; ii) a substrate with surface-attached probes (such as an SHG-labeled, SFG-labeled or DFG-labeled target protein); and iii) a detector for measuring the intensity of the second harmonic or other nonlinear optical beams. In some examples of the system, binding of an agent to an allosteric site on a protein is detected by the apparatus and data recorded by the detector can be recorded on a fixed or data storage medium that is accessible via a system for reading the storage medium.

In other examples, the system further comprises a monochromator (for wavelength selection), a pass-filter, color filter, interference or other spectral filter (for wavelength selection or to separate the fundamental(s) from the higher harmonics), one or more polarizing optics, one or more mirrors or lenses for directing and focusing the beams, computer control, or software. The system can comprise a computer having a processor, the processor operatively coupled to a memory, and configured to implement the computer control or software (e.g., to execute algorithm(s) embodying the software). In other examples, the mode of delivering or generating the nonlinear optical light (e.g., SHG) can be based on one or more of the following means: TIR (total internal reflection), fiber optics (with or without attached beads), transmission (fundamental passes through the sample), reflection (fundamental is reflected from the sample), scanning imaging, confocal imaging or scanning, resonance cavity for power build-up, or multiple-pass set-up.

In another example of the system, measured information can take the form of a vector which can include one or more of the following parameters: intensity of light (typically converted to a photovoltage by a PMT or photodiode), wavelength of light (determined with a monochromator and/or filters), time, or position. Two general configurations of the apparatus can be image scanning (e.g., imaging of a substrate-intensity, wavelength, etc. as a function of x,y coordinate) and spectroscopic (e.g., measurement of the intensity, wavelength, etc. for some planar surface or for a suspension of cells, liposomes or other particles).

The invention can be further understood by reference to the following examples, which are provided by way of illustration and are not meant to be limiting.

### EXAMPLES

### EXAMPLE 1: METHOD OF SCREENING DRUG CANDIDATES TARGETING ALPHA SYNUCLEIN

Here we report on the first use of SHG as a ligand screening method, using a readout of conformational change of the target protein in real time. We focused on spermine-induced conformational change of the protein alpha-synuclein, a therapeutic target for Parkinson's disease (PD). We asked whether we could identify a ligand that binds to the protein and modulates its conformation, using spermine as a tool compound. Alpha-synuclein is a 140-residue highly acidic protein and is a major target for PD. PD is the second most common neurodegenerative disease after Alzheimer's disease and affects between 1-1.5 million people in the U.S. (Lang AE & Lozano AM, New England Journal of Medicine 339(16):1130-1143, 1998). The two hallmarks of the disease are the loss of dopaminergic neurons in the substantia nigra of the midbrain and the presence of Lewy bodies (LBs) in neurons, comprised mainly of aggregates of alpha-synuclein. Point mutations in alpha-synuclein are related to rare forms of early-onset PD, suggesting that the protein is implicated in the disease mechanism (Klockgether T, Cell and Tissue Research 318(1):115-120, 2004). Growing evidence suggests that oligomers of alpha-synuclein, formed prior to the larger aggregates found in the brains of patients with the disease, may be neurotoxic *in vivo* (Winner B, et al., Proceedings of the National Academy of Sciences. 108(10)): 4194-4199, 2011). The process by which the protein proceeds from monomer to fibril aggregates is currently the subject of intense scrutiny (Norris EH, et al., Journal of Biological Chemistry 280(22):21212-21219, 2005; Karpinar DP, et al., EMBO J 28(20):3256-3268, 2009). Recent reports indicate that the protein adopts a tetrameric structure *in vivo,* implying that dissociation to the monomer precedes oligomerization and aggregation. Regardless of the toxic species, monomeric alpha-synuclein is an attractive therapeutic target for a drug which can stabilize it and prevent aggregation at the earliest stages. Alpha synuclein has been studied extensively *in vitro* (Winner B, et al., Proceedings of the National Academy of Sciences. 108(10)): 4194-4199, 2011; Bertoncini CW, et al., Proceedings of the National Academy of Sciences of the United States of America 102(5): 1430-1435, 2005; Conway KA, Harper JD, & Lansbury PT, Biochemistry 39(10):2552-2563, 2000). It is natively unstructured, adopting a range of conformations from compact to fully extended (Bertoncini CW, et al., Proceedings of the National Academy of Sciences of the United States of America 102(5):1430-1435, 2005). Interactions between the C- and N-termini appear to stabilize the protein predominantly in a compact conformation. One hypothesis states that when the protein is exposed to polyamines such as spermine or to low pH these interactions are disrupted and the rate of aggregation *in vitro* greatly increases. Another hypothesis for spermine's effect states that bound spermine reduces the highly negative charge state of the C-terminal region, lowering the electrostatic repulsion between protein molecules and leading to the greatly enhanced aggregation rate (McClendon S, Rospigliosi CC, & Eliezer D, Protein Science 18(7):1531-1540, 2009). In either case, the protein undergoes a conformational change preceding aggregation.

Toward the aim of identifying binders or conformational modulators of alpha-synuclein, we built an SHG-based screening instrument called Artemis which reads templated microscope slides. The effect of a ligand on the protein's conformation is detected and reported by the instrument in real time. A silicone template defines 16 wells on each slide; measurement of a well takes seconds to complete. Current throughput is on the order of hundreds of assays per day, suitable for fragment or focused library screening, but we intend to scale the technique to high throughput by implementing standard fluidics and automation.

We developed an SHG assay of conformational change for the natively unstructured protein, monomeric alpha-synuclein, a target of Parkinson's disease (PD). Alpha-synuclein aggregation from monomer to oligomers to fibrils is implicated in pathogenesis and is thought to be preceded by a change in monomer conformation induced by binding to a ligand or a change in pH. Spermine, an endogenous polyamine, induces a conformational change in the protein that increases its aggregation rate *in vitro* by a factor of 10⁵ and may play a role in pathogenesis. We exploited spermine as a tool compound in constructing a conformational change assay of alpha synuclein. We screened a fragment library using this assay for compounds that could inhibit spermine's effect on the protein's conformation. Our screen identified one compound from the library that completely abolishes spermine's effect. When we tested its effect on the protein's conformation on its own, the compound produces an SHG response 'signature' of conformational change similar to spermine's, suggesting that it may induce the same conformation and bind to the same site as spermine. We subsequently confirmed by ¹H, ¹⁵N heteronuclear single-quantum coherence (HSQR) NMR that the compound binds to the C-terminal region of the monomer protein, the region where spermine binds. Few compounds are known to bind to alpha-synuclein because the protein has been difficult to screen by conventional techniques, so our method offers a means to identify compounds that can stabilize non-aggregating conformations of the monomer, as well as analogous ones for beta-amyloid, a target of Alzheimer's disease. The compound we discovered is the first known to bind to the C-terminal region of the protein and could be a useful starting point for development of therapeutic leads. As SHG can be applied in principle to any target, the technique we introduce here provides a sensitive and rapid means to identify and validate ligands that modulate the conformation of the protein, with broad potential for basic research and drug discovery.

### Methods

*Alpha-Synuclein Purification and Labeling:* A single-site cysteine mutant (A90C) of the alpha-synuclein was constructed and labeled with PyMPO-maleimide (Invitrogen), an SH-active probe that we have used in previous studies (Salafsky, Journal of Chemical Physics 125(7), 2006; Salafsky, Physical Chemistry Chemical Physics 9(42):5704-5711, 2007). Mutant (A90C) protein was previously determined to function like wild-type (Bertoncini CW, et al., Proceedings of the National Academy of Sciences of the United States of America 102(5): 1430-1435, 2005). The label was coupled to the protein according to the manufacturer's instructions and purified by gel filtration.

*Artemis SHG Screening Instrumentation:* The Artemis instrument is comprised of a mode-locked Ti:sapphire oscillator that provides the fundamental beam necessary to generate second-harmonic light (high peak power intensity). We used a Mira 900 Ti:Sapphire ultrafast oscillator (Coherent Inc.) pumped by a Millenia V DPSS laser (Spectra-Physics Corp.) as the fundamental source. The Ti:sapphire oscillator produced 0.75W of fundamental power at 780 nm with pulse duration of about 200 fs at 80 MHz. The fundamental was passed through a half-wave plate to select p-polarization for all the experiments described in this example, and focused into a Dove prism for total internal reflection (TIR) to a spot size of ∼50 µm. The second harmonic light was collected by a condenser lens, separated from the fundamental using a dichroic mirror and wavelength filters, and directed into a PMT module with a built-in pre-amplifier for photon counting (Hamamatsu). A custom electronics board was used to digitize the signal and the data was sent to a computer running the control and data collection software (LabView).

The slide with protein is coupled to a prism using BK7 index matching fluid (Cargille) and the prism itself secured onto a 1-D translation stage capable of 10 µm randomly-addressable precision (Renishaw). Each of the 16 wells is read in sequence by translating the stage via the control software. Artemis was outfitted with a single-channel liquid injector (Cavro) which delivered a liquid bolus. The liquid bolus volume and delivery speed could be varied. In the experiments described here, we used a volume of 10 µl delivered in 1 second. The measured parameters include the read time before and after ligand injection, integration time, stage temperature (kept at room temperature for all experiments here), and the stage position, relative to the line of the fundamental beam.

*Alpha-synuclein Assay Slide Preparation:* A custom silicone template with adhesive backing (Arrowleaf Research) was applied to commercially available Xenobind glass microscope slides (Xenopore) using a custom machined assembly jig to define 16 wells of diameter 3 mm (volume ∼15 µl) on each slide. The template defining the wells is registered to a standard 96-well plate. Stock solutions of PyMPO-Maleimide labeled alpha-synuclein were diluted to 5 µM final concentration in Phosphate Buffered Saline (PBS) and allowed to adsorb to the glass surface in each well by incubating at room temperature for 30 minutes. After allowing the protein to absorb, the slide was placed on the Artermis platform and each well was scanned via SHG to determine the amount of protein in each well. Unbound protein was then removed by washing five times with 20µL PBS. A post-wash well scan was then performed to determine how much protein was bound to the slide surface.

*Maybridge Library Screening:* The Maybridge Ro3 fragment library contains over 1,000 pharmacophoric compounds of ≥95% purity solubilized at 50 mM in DMSO. For fragment screening experiments, a single compound from the library was diluted in PBS and placed in each well at a final concentration of 1mM (4% DMSO). To ensure that a buffer mismatch did not occur, the wash buffer used during screening was PBS + 4% DMSO. The slide was then loaded on to the instrument, baseline signal was measured in real time, and spermine was injected at 5 mM while reading the SHG signal. In these experiments, baseline signals were monitored for 8 seconds, followed by spermine injection and recording the SHG signal was recorded for an additional 8 seconds.

*Dose-Response Curve Fitting:* To determine the dose-response curves of Spermine (Sigma), Spermidine (Sigma), and COMPOUND A, serial dilutions of each compound were used to prepare stock solutions. Each compound was added to a well to produce the indicated final concentration. For Spermine, the range of concentrations tested was 10µM, 200µM, 400µM, 600µM, 800µm, 1mM and 5mM. For Spermidine the concentrations were 10µM, 62.5µM, 125µM, 250µM, 500µM, 1mM, and 30mM. For COMPOUND A the concentration range was 5µM, 32.5µM, 62.5µM, 125µM, 250µM, 500µM, and 1mM. The concentrations were then converted to the log scale and the data plotted in GraphPad Prism V5 using the non-linear regression fit for log(agonist) vs response with variable slope. The data were normalized so that the value for the lowest concentration and the highest concentration equaled 0 and 100%, respectively. The EC₅₀ values were calculated by the software.

*Competition Assays:* For the Spermine-Spermidine competition assays, a final concentration of 1mM Spermine was placed into each well containing 5µM labeled alpha-synuclein and the change in SHG intensity was measured. The protein was then allowed to incubate in the presence of 1mM spermine for 25 minutes at room temperature. After 25 minutes, 3mM spermidine was added to each well and the resulting change in SHG intensity was measured. The Analog-Spermine competition assays were performed as above except that each individual analog was first incubated with alpha-synuclein for 25 minutes and then 1mM spermine was added to the well.

For the Spermine-COMPOUND A competition assay, 5mM Spermine was placed into each well containing 5µM labeled alpha-synuclein and the change in SHG intensity was measured. The protein was allowed to equilibrate for one minute in the presence of spermine, and either 100µM or 1mM COMPOUND A was added to each well and the change in SHG intensity was measured.

*Quantifying Changes in SHG Intensity:* Control experiments were performed on each compound to empirically determine the time frame required for each compound to produce a maximal response (Tₘₐₓ). For all experiments except those using Phthalocyanine Tetrasulfonate (PcTs; Sigma), the maximal response as measured by SHG occurred within 30s after compound addition. For the PcTs experiments, the maximal response occurred at approximately 45s after compound addition.

To calculate the percent change, the SHG intensity as measured just prior to injection (T₀) was subtracted from the SHG intensity at Tₘₐₓ and then divided by T₀ according to the following equation: SHG% = (I_{SHG}@Tₘₐₓ - I_{SHG}@T₀)/I_{SHG}@T₀.

A control buffer injection was used to determine the threshold for SHG intensity change and was calculated in a similar manner to above. The net SHG intensity change for each compound was then reported by subtracting the buffer threshold value from the SHG intensity value produced by compound addition. The resulting value was reported as the percent change in SHG intensity due to compound addition.

### Results

*Spermine Induces a Conformational Change in Alpha-Synuclein*: In order to screen for novel compounds that inhibit alpha-synuclein conformational change by Second Harmonic Generation (SHG), we developed an assay using spermine. Spermine is a polyamine involved in numerous cellular processes and has previously been shown to induce a conformational change in alpha-synuclein that results in an increased aggregation rate *in vitro.* In our assay, each well of a custom assembled microscope slide was loaded with 5 µM alpha-synuclein, a concentration below the threshold for oligomer formation (Fernandez CO, et al., EMBO J 23(10):2039-2046, 2004), and SHG signals before and after spermine addition were measured in real time. Upon addition of 5 mM spermine, the SHG signal intensity instantly increases (**Figure 1A**). We measured the change in the SHG intensity to be 36.26±4.97%. This change corresponds to a known conformational change induced by spermine binding to the C-terminus of alpha synuclein as shown by NMR (Bertoncini CW, et al., Proceedings of the National Academy of Sciences of the United States of America 102(5):1430-1435, 2005). Using SHG, we obtained a dose-response curve for spermine binding to alpha-synuclein (**Figure 1B**), and determined spermine's EC₅₀ to be 0.84 mM (logEC₅₀ -3.07) [repeated: 0.79 mM (logEC₅₀ -3.13)].

Spermidine, a polyamine closely related to spermine, is also known to bind alpha-synuclein and induce conformational change. As an additional control, we tested its effect on the protein and found it also induces a conformational change in alpha-synuclein with an SHG signature similar to spermine's (**Figure 1C**). A 3 mM dose of spermidine results in a 19.88 ± 2.37% increase in SHG intensity. We next determined spermidine's dose-response curve (**Figure 1D**). By SHG, we measured spermidine's binding affinity to be 1.11mM (logEC50 = -2.95 ± 0.19). To verify the EC₅₀ results of our SHG derived dose response curves, we employed a competition assay between spermine and spermidine. In the competition assay (**Figure 1E**), 1mM spermine was added to wells containing alpha-synuclein and a 25.58 ± 4.33 % (repeated: 30.23 ± 3.69 %) increase in SHG intensity was measured. After incubation of the alpha-synuclein in the presence of 1mM Spermine for 25 minutes, 3mM Spermidine was added to the sample and a 5.1 ± 0.81% change in SHG intensity was recorded. Given that 3 mM spermidine addition alone induces a ∼20% increase in SHG intensity, the relatively small (∼5%) increase seen in the presence of spermine suggests that spermine binds with a higher affinity to alpha-synuclein than spermidine, in agreement with our experimentally derived dose-response curves.

*Second Harmonic Generation as a Platform for Molecular Screening:* Having demonstrated that the SHG signal change we observe corresponds to spermine- or spermidine-induced conformational change, we sought to screen for novel compounds that would inhibit the spermine induced conformational change of alpha-synuclein. Using an SHG based competition assay, we screened in duplicate over 1000 ligands from the Maybridge Rule of Three (Ro3) fragment library for their ability to inhibit alpha-synuclein's conformational change upon spermine addition. Each well was loaded with 5 µM alpha-synuclein and pre-incubated with one fragment compound from the Maybridge Ro3 library for 30 minutes at 1 mM in assay buffer. Spermine was then added to each well at 5 mM and the resulting change in SHG signal was measured. Only one compound from the entire library of 1,000 compounds, COMPOUND A, completely prevented spermine-induced conformational change at a concentration of 1 mM as measured by SHG (-1.39±1.05; **Figure 2A**). Interestingly, COMPOUND A, shown in **Figure 2B****,** bears a resemblance in charge and length to spermine. As shown in **Figure 2C** (right trace), addition of 1mM COMPOUND A to alpha-synuclein changes the SHG baseline signal immediately. We measured the change in SHG intensity to be 31.67±4.31% and classified its SHG response using direction (relative to baseline), magnitude and rate as similar to spermine's response (**Figure 3D**).

Next, we determined if COMPOUND A was able to induce a conformational change in alpha-synuclein as measured by SHG. As shown in **Figure 3A****,** addition of 1mM COMPOUND A to alpha-synuclein changes the SHG baseline signal immediately. We measured the change in SHG intensity to be 30±2% (repeated: 31.67±4.31%) with the direction (relative to baseline), magnitude and kinetics similar to spermine's response. This result suggested that COMPOUND A induces a conformational change in alpha-synuclein as similar that of spermine. We next titrated COMPOUND A and measured its dose-response by SHG (**Figure 3B**). From this, we determined the ECso to be 97.8µM (logEC₅₀ -4.01 ± 0.28). With a K_{d} of approximately 100µM, COMPOUND A has an approximately 5.5 to 8 fold higher affinity for alpha-synuclein than that of spermine.

Given that COMPOUND A has a much higher affinity for alpha-synuclein than spermine, COMPOUND A should outcompete spermine for binding to alpha-synuclein. To determine if COMPOUND A can outcompete spermine for binding to alpha synuclein, we performed a competition assay in which 5mM spermine was added to alpha-synuclein containing wells and allowed to equilibrate for 1 minute. Addition of spermine alone resulted in a 37.44 ± 6.40% increase in SHG intensity (**Figure 3C**). After incubation of alpha-synuclein in the presence of 5mM spermine, either 1mM or 100µM COMPOUND A was added to each well and the change in SHG intensity was recorded. The addition of 1mM COMPOUND A to alpha-synuclein pre-bound to spermine resulted in a 40.69 ± 5.81% increase in SHG intensity. This is in contrast to the addition of 100µM COMPOUND A, which resulted in a 3.18 ± 1.50% increase in SHG intensity. These data demonstrate that COMPOUND A outcompetes spermine for binding to alpha-synuclein due to a higher affinity for its target. Further comparisons are shown in **Figure 3D****.**

The results from our SHG experiments indicated that COMPOUND A bound to alpha-synuclein and induced a conformational change similar to spermine binding but with a higher affinity for alpha synuclein. We next wanted to determine if COMPOUND A bound to the same site on alpha-synuclein as spermine. To do this we turned to an independent structural technique 2-D HSQR NMR, to determine the structure of COMPOUND A in complex with alpha-synuclein.

The combination of our SHG and NMR data demonstrate that COMPOUND A binds to alpha-synuclein in the same site as spermine (**Figure 6A**) and with a higher affinity, thereby verifiying the results of our initial screen as well as validating SHG as a structural screening technique.

The interaction between alpha-synuclein and COMPOUND A was analyzed by NMR spectroscopy. NMR resonances are highly sensitive probes of protein-protein and protein-ligand interactions and therefore can provide a detailed description of interaction interfaces and binding affinities. Compound A was added to ¹⁵N-labeled alpha-synuclein and changes in chemical shifts were followed in two-dimensional ¹H-¹⁵N correlation spectra of alpha-synuclein (**Figure 6B**). Significant changes in NMR signals were observed for residues G111 to A140 of alpha-synuclein (**Figure 6B** and **Figure 6C**). A very similar perturbation profile had been observed upon addition of spermine to alpha-synuclein, indicating that both COMPOUND A and spermine bind to the C-terminal domain of alpha-synuclein.

The consequences of binding of COMPOUND A onto the ensemble of conformations populated by alpha-synuclein in solution was studied using paramagnetic relaxation enhancement (PRE). The interaction between a specifically attached paramagnetic nitroxide radical and nearby protons (less than ≈25 Å) causes broadening of their NMR signals because of an increase in transverse relaxation rate. This effect has an *r*⁻⁶ dependence on the electron-proton distance and thus allows the detection of long-range interactions in proteins. To observe transient long-range contacts within alpha-synuclein in the presence of COMPOUND A, we attached a paramagnetic MTSL label at position 90 in alpha-synuclein. A comparison of the PRE profiles of MTSL-A90C alpha-synuclein in the absence and presence of COMPOUND A revealed decreased paramagnetic broadening at residues 40-46 and the C-terminal region covering residues 124-136 (**Figure 6D**). Thus, binding of COMPOUND A attenuates transient long-range interactions in alpha-synuclein. Attenuation of long-range interactions in alpha-synuclein have also been reported upon binding to spermine, in combination with a spermine-induced acceleration of the aggregation of alpha-synuclein . Therefore, COMPOUND A was further tested for enhancement of the aggregation of alpha-synuclein. In agreement with the similar binding mode identified by SHG and NMR, COMPOUND A also significantly shortened the lag phase of alpha-synuclein fibrillization (**Figure 7A**). The morphology of alpha-synuclein fibrils obtained in presence (**Figure 7B**, left) and absence (**Figure 7B**, right) of COMPOUND A was similar. The combination of our SHG and NMR data thus demonstrates that COMPOUND A binds to the alpha-synuclein C-terminus. In addition, the NMR and PRE data indicate that COMPOUND A binds to the same site as spermine, or to one with substantial overlap with it, and affects the protein's conformation by attenuating the long-range interactions in a similar manner as observed for spermine thereby confirming the results of the initial screen and validating SHG as a structural screening technique.

*COMPOUND A is a Selective Inhibitor of Spermine's Ability to Induce Conformational Change in Alpha-Synuclein*: We identified only one compound in our screen using the Maybridge Ro3 library using the hit criteria of abolishment of spermine's response (e.g., within 1% of baseline post-addition) at a fragment concentration of 1 mM. To explore the effects of chemical analogs of COMPOUND A from our library under less stringent conditions, we used the program Acelot SimFinder (acelot.com/SimFinder.html), to rank the top five compounds. As seen in **Figure 4A****,** the analogs are similar to COMPOUND A structurally and chemically. We first determined whether any of these analogs would, by themselves, induce a change in SHG intensity when exposed to alpha-synuclein. The addition of each individual analog at 1mM resulted in an increase in SHG intensity (**Figure 4B**; **Table 1**). Although the increase in SHG intensity indicates a conformational change in alpha-synuclein, the magnitudes of the SHG signal changes are significantly lower than the values reported for both spermine and COMPOUND A (t-test). As the SHG responses of these changes are similar in amplitude and direction relative to baseline, the lower magnitudes may indicate a lower potency for causing the same conformational change rather than a conformational change different from the one induced by COMPOUND A. For example, the alpha-synuclein targets may be labeled the same way, and the conformational change induced by different binding entities may occur in different locations on or within each alpha-synuclein target, and thus at different distances from the label. Change(s) in conformational state of the alpha-synuclein may correlate to a known pharmacological property or a known functional effect. In some cases, the correlation may be specific to conformational change in a given location on the alpha-synuclein. Therefore, conformational change occurring away from the label location may not be relevant the known pharmacological property or known functional effect assayed.

A similar comparison to **Figure 4B** is shown in **Figure 4D****,** where COMPOUND A is shown alongside the 5 analogs.

To better understand the results of the COMPOUND A analog data, we tested Analogs 1, 2 and 5 for binding to alpha-synuclein by NMR. The analysis of chemical shift differences as seen in **Figure 4E** suggest that Analogs 1 and 2 bind alpha-synuclein more weakly than COMPOUND A.

**Table 1: Quantification of COMPOUND A Analog Data**

| Compound | Compound Only (% Shift / % Change) | Competition Assay (% Shift / % Change) |
|---|---|---|
| Spermine | | N/A |
| 5mM | 37.44 ± 6.40% (repeated: 36.26±4.97%) | |
| 1mM | 30.23 ± 3.69% | |
| COMPOUND A (1mM) | 40.69 ± 5.81% (repeated: 31.67±4.31%) | -1.39 ± 1.05% |
| Analog 1 | 5.96 ± 0.81% | 15.79 ± 5.06% |
| Analog 2 | 8.30 ± 0.37% | 14.50 ± 1.94% |
| Analog 3 | 6.46 ± 0.28% | 9.23 ± 1.74% |
| Analog 4 | 7.44 ± 0.78% | 12.30 ± 1.88 |
| Analog 5 | 11.24 ± 2.72% | 14.09 ± 4.78 |

Next, we performed a competition assay in which each of the analogs was incubated in the presence of alpha-synuclein for 25 minutes. After 25 minutes, 1mM spermine was added to each well and the change in SHG intensity recorded. The addition of spermine to alpha-synuclein incubated in the presence of analogs resulted in an increase in SHG intensity for all analogs (**Figure 4C****; Table 1**). These data suggest that while chemically and structurally similar to COMPOUND A, the analogs have lower ability to induce alpha-synuclein aggregation and a lower affinity for alpha-synuclein than both spermine and COMPOUND A. Additionally, the results from our analog competition assay confirm that COMPOUND A is the only competitive synuclein aggregator from the Maybridge Ro3 library, thus validating our initial screen.

*Phthalocyanine Tetrasulfonate Produces a Distinct SHG Signature*: Our data on COMPOUND A suggest that it binds in the same site on alpha-synuclein as spermine, producing a conformation that is known to increase the aggregation rate of alpha-synuclein *in vitro.* Because increased aggregation of alpha-synuclein is linked to pathogenesis of neurodegenerative diseases, we wanted to determine if a known inhibitor of alpha-synuclein aggregation, Phthalocyanine Tetrasulftonate (PcTs), produced a distinct SHG signature when compared to spermine. As shown in **Figure 5A****,** we observed an 83.74 ± 6.41% (repeated:-79.6 ± 2.7%) decrease in the SHG intensity of alpha-synuclein upon treatment with 10µM PcTs. Additionally, the SHG intensity trace (**Figure 5A**) for PcTs treatment produces a completely unique SHG signature when compared to other molecules that induce the aggregation prone conformation of alpha-synuclein. As shown in **Figure 5B****,** the change in the SHG intensity of alpha-synuclein was measured to be -79.6 ± 2.7% upon treatment with 10 µM PcTS.

These data suggest that novel inhibitors of alpha-synuclein aggregation can be identified by selecting compounds that produce an SHG signature similar to PcTs.

Finally, Compound A was tested in a Thioflavin T assay (**Figure 7A**) and found to increase the rate of aggregation of alpha-synuclein *in vitro* by roughly two-fold, the same increase seen in the presence of spermine. Therefore, the compound has about the same functional effect as spermine. As discussed above, the compound also induces the same conformational change as spermine.

### Discussion

A key limitation in drug discovery is the ability to efficiently search chemical space to identify ligands that induce conformational change in clinically important target proteins. In the work described here, we present the first example of using Second Harmonic Generation as a structural technique for the identification of conformational modulators. We screened a fragment library containing over 1,000 compounds and identified a single compound that inhibited the spermine induced conformational change of alpha-synuclein. That compound, COMPOUND A, was shown to both cause a conformational change on its own and bind with a higher affinity to alpha-synuclein than spermine, suggesting that the ligand caused the same conformational change as spermine. We then used NMR to demonstrate that COMPOUND A bound to alpha-synuclein at the same site as spermine and induced a similar conformational change.

*Second Harmonic Generation as a New Platform for Drug Discovery*: This work demonstrates SHG to be a powerful method for determining the mechanism of ligand action, particularly if known conformational modulators such as spermine are available for comparison purposes. Two classes of ligands can potentially be found by our competition-based screen, classified by mechanism of action in preventing spermine-induced conformational change of alpha-synuclein. The ligands in the first class are steric inhibitors that bind at or near the spermine binding site. The ligands in the second class are allosteric inhibitors that induce a conformational change in alpha-synuclein that prevent spermine binding. The NMR data presented in this work confirms that COMPOUND A is a member of the first class of inhibitors.

More surprising, however, was the fact that we identified only one compound from the Maybridge Ro3 library. While spermine and COMPOUND A are similar in length and charge, a number of compounds in the library share structural similarities with COMPOUND A. These minor structural differences include the conversion of a 5-membered ring in COMPOUND A to a 6-membered ring and the presence of additional side chains. The ability of SHG to identify COMPOUND A as a specific and potent inhibitor of alpha-synuclein's spermine induced conformational change as well as distinguish it from closely related molecules in our assays demonstrates the sensitivity of the technique and demonstrates the effectiveness of SHG as a screening platform.

*SHG Signatures as Tools for Molecular Screening*: Phthalocyanine Tetrasulfonate (PcTs) is a known inhibitor of alpha-synuclein aggregation *in vitro.* Despite its anti-aggregation effects, PcTs is not a clinically relevant compound for treatment of neurodegenerative disease. However in our assay, it has the potential to be useful for the identification of novel compounds which inhibit alpha-synuclein aggregation. This is due to the fact that each molecule produces a "SHG signature" in direction of signal change relative to baseline, magnitude of change and kinetics. These signatures provide a more stringent means of classifying a ligand than binding information alone. Additionally, by comparing a molecule's signature to one produced by a known modulator, we can make putative classifications of that molecule's mechanism of action (under the same labeling and tethering conditions). We can also screen compound libraries by SHG signature in an effort to identify compounds that produce similar signatures to known conformational modulators. For example, this approach can be used to screen the Maybridge Ro3 library for potential anti-aggregation compounds which induce a similar conformational change in alpha-synuclein as PcTs.

### EXAMPLE 2: DIFFERENTIAL SCREENING OF DRUG CANDIDATES TARGETING ALPHA SYNUCLEIN

COMPOUND A and its analogs (**Figure 4A**) comprise a library of 6 structurally (and chemically) similar candidate biochemical entities. The compound only (% shift or change) SHG responses of COMPOUND A and its analogs in **Table 1** can be used to perform a differential screen. COMPOUND A and each of the analogs induce a change in SHG intensity when exposed to alpha-synuclein. Each of the candidate biochemical entities generated a distinct SHG signature. The addition of COMPOUND A at 1mM resulted in an increase in SHG intensity (**Table 1**). The addition of each individual analog at 1mM also resulted in an increase in SHG intensity (**Figure 4B****; Table 1**). The signature responses in **Table** 1 were generated by measuring a first conformational state of the alpha-synuclein, thereby generating an SHG baseline, contacting each of the 6 structurally similar candidate biochemical entities with the alpha-synuclein, and measuring a second conformational state of the alpha-synuclein, thereby generating an SHG signature for the 6 candidate biochemical entities. The SHG signatures each indicate a change in conformational state of the alpha-synuclein (all 6 signals show an increase in SHG intensity). Further, each of the candidate biochemical entities produces a different change in conformational state of the alpha-synuclein (target biochemical entity).

The SHG signatures in **Table 1** can be compared with each other (e.g., to determine similarity). For example, the SHG signatures can be analyzed using a computer-executable program. In some cases, consecutive SHG signatures in a time series can be compared, and the next SHG signature can be compared against a biochemical entity selected based on the comparison in the previous step. In some cases, any two or more of the SHG signatures can be compared. In some cases, the SHG signatures of all 6 of the biochemical entities in the library can be compared against each other. In some cases, the SHG signatures can be compared against the SHG signature with a highest, lowest or otherwise defined reference value. The differential screen described herein may be used, for example, in situations where no known modulator (e.g., approved drug) exists (e.g., for some intrinsically disordered proteins). In some examples, consecutive or simultaneous comparisons may be made in order to select a biochemical entity among the candidate biochemical entities.

In this example, the SHG signature was generated by measuring a change in magnitude of the SHG signal (e.g., end-point change in conformational state of the target biochemical entity; alternatively, a kinetic (real time) change in conformational state of the target biochemical entity can be measured) relative to the SHG baseline, as well as a change in direction of the SHG signal relative to the SHG baseline (SHG parameters). While different, in some cases, at least a subset of the SHG signatures of the 5 analogs may be classified (pairwise or all) as similar to each other using one or more SHG similarity parameters. For example, a difference in change in magnitude of the SHG signal relative to the SHG baseline is within 50% of each other for the 5 analogs and a difference in change in direction of the SHG signal relative to the SHG baseline is the same for all 6 biochemical entities in the library.

### EXAMPLE 3: SHG DETECTION OF APPROVED DRUG TARGETING ALPHA SYNUCLEIN

In this example, alpha-synuclein causes a signal change upon exposure to dopamine, a metabolite of its pro-drug Levodopa (an approved drug). Levodopa, also called L-dopa, which is converted to dopamine in the brain, remains the gold standard for treating Parkinson's disease. Abnormal folding and aggregation of alpha-synuclein is thought to be the major causative agent of the disease. Levodopa is the most effective treatment for the symptoms associated with Parkinson's disease. However, levodopa is a prodrug (i.e., precursor chemical compound of a drug) and a precursor to dopamine, which is the biologically active form of the drug.

### Experimental Conditions

An alanine to cysteine mutation (A90C) was introduced into alpha-synuclein to produce a site-specific peptide for dye conjugation, as native alpha-synuclein lacks cysteine residues. A90C alpha-synuclein was then labeled with PyMPO-Maleimide using standard techniques and the location of the modified peptide was verified by mass spectroscopy to be cysteine 90.

For SHG experiments performed with alpha-synuclein, a stock solution of PyMPO-Maleimide-labeled A90C alpha-synuclein was diluted to a final concentration of 5µM in 50mM Hepes, pH 7.4, 100mM NaCl. The 5µM protein solution was then immobilized onto an aldehyde derivatized glass slide by incubating for one hour at room temperature. Control (buffer) and compound injections were performed in 50mM Hepes, pH 7.4, 100mM NaCl.

The experiments were performed with an instrument comprising a mode-locked Ti:sapphire oscillator which provides the fundamental beam necessary to generate the second-harmonic signal (high peak power intensity). We used a Mira 900 Ti:Sapphire ultrafast oscillator (Coherent Inc.) pumped by a Millenia V DPSS laser (Spectra-Physics Corp.). The Ti:sapphire oscillator produced ∼0.7 W of fundamental power at 780 nm with pulse duration of ∼150 fs at 80 MHz. The fundamental was passed through a half-wave plate to select p-polarization (used for all the experiments in this example), and focused into a Dove prism for total internal reflection (TIR) to a spot size of ∼50 µm. The second harmonic light was collected by a condenser lens, separated from the fundamental using a dichroic mirror and wavelength filters, and directed into a PMT module with a built-in pre-amplifier for photon counting (Hamamatsu). A custom electronics board was used to digitize the signal and the data was sent to a computer running the control and data collection software (LabView).

### Results

**Figure 8** and **Figure 9** show the effect of dopamine treatment upon alpha-synuclein conformation.

**Figure 8** shows an example of a kinetic (real time) change in SHG intensity of alpha-synuclein upon addition of Dopamine. Addition of 1mM Dopamine (indicated by the arrow in **Figure 8**) results in an increase in the (normalized) SHG intensity of alpha-synuclein over the pre-injection level (at times before the injection marked by the arrow). The change in SHG intensity is the result of a change in the conformation of alpha-synuclein upon dopamine binding.

**Figure 9** is a quantification of an average change in SHG intensity of alpha-synuclein upon addition of either buffer alone (left) or 1mM Dopamine (right). The average percent change in the SHG intensity of alpha-synuclein upon control (buffer) injection is-1.74 ± 0.57%. Error bars = SEM, N= 4. The average percent change in the SHG intensity of alpha-synuclein upon injection with 1mM Dopamine is 3.375 ± 1.13%. Using an unpaired t-test, the P value (indicated by ** in **Figure 9**) is determined to be statistically significant (P value = 0.0067).

### Discussion

The SHG results in **Figure 8** and **Figure 9** (e.g., kinetic or end-point SHG signatures, signals or signal differences) may be used to identify novel drug candidates (e.g., by comparing the SHG results for dopamine with SHG results for the candidate drugs) to substitute or improve upon the active form of the approved drug by, for example, selecting compounds that produce an SHG signature similar to dopamine. These alternative drugs may exhibit similar or improved *in vivo* and *in vitro* pharmacological properties, phenotype and/or mechanism of action compared to the active form of the approved drug. For example, if a drug candidate exhibits the same SHG response as dopamine, it may exhibit a substantially similar therapeutic efficacy and/or pharmacological (or pharmacokinetic) properties. In some cases, such novel drug candidates may exhibit improved toxicity and/or clinical safety properties. Illustrative examples of *in vivo* and *in vitro* pharmacological properties, such as pharmacokinetic profile, changes in phenotype, efficacy and other clinical data, etc., of levodopa are set forth in David J Brooks, "Optimizing levodopa therapy for Parkinson's disease with levodopa/carbidopa/entacapone: implications from a clinical and patient perspective," Neuropsychiatr Dis Treat. Feb 2008; 4(1): 39-47*,* and references therein.

### EXAMPLE 4: DETERMINATION OF STRUCTURE-ACTIVITY RELATIONSHIP (SAR)/ CATALOGING

A library comprising 200 distinct compounds and Sorafenib, each with a molecular weight between 400 and 500 g/mol, three aromatic groups, and 2-5 nitrogen atoms, is evaluated for the structure-activity relationship using a surface array comprising more than 200 copies of a Raf kinase. The conformation state of the Raf kinase is measured by single-harmonic generation (SHG) methods described in the present application to establish a baseline in the absence of any compound. Each compound in the library is then contacted with a single copy of the Raf kinase on the array. The conformational state of each copy of the single kinase target is measured by SHG methods. The SHG signature for each compound is determined based on the change in conformational state upon contacting the Raf kinase. Sorafenib has a SHG signature with a 30% shift (i.e. magnitude) in the positive direction. The compounds are grouped, based on their SHG signature and common structural parameters, to generate a structure-activity relationship (SAR) catalog. For example, the 8 compounds that have a signature within 10% in magnitude to that of Sorafenib are cataloged as "molecular weight between 400 and 500 g/mol, three aromatic groups, 2-5 nitrogen atoms, and a signature with +27% to +33% magnitude". Based on this SAR profile, the compounds are further tested for their inhibitory activity against the Raf kinase, and it is discovered that three of the compounds indeed exhibit anti-Raf activity at nanomolar concentrations. As such, the SHG signature can provide an addition means of assaying the properties of a compound, wherein the compound can be classified based on structure-activity relationships using the SHG signature.

## Claims

1. A method of screening biochemical entities, the method comprising:
(a) providing a library comprising 6 or more candidate biochemical entities;
(b) measuring a first conformational state of an intrinsically disordered protein, thereby generating a baseline;
(c) contacting each of the candidate biochemical entities with the same intrinsically disordered protein;
(d) measuring a second conformational state of the intrinsically disordered protein;
(e) determining a signature for at least a first and a second candidate biochemical entity using the second conformational state
(f) comparing the signature of the first candidate biochemical entity to the signature of the second candidate biochemical entity; and
(g) selecting, based on the comparison in (f), one or more biochemical entities from the candidate biochemical entities,
wherein the candidate biochemical entities are small molecules that share a common scaffold core, and wherein the measuring steps comprise measurements of second harmonic generation (SHG).

2. The method of claim 1, wherein, each of the candidate biochemical entities produces a different change in conformational state of the intrinsically disordered protein.

3. The method of any one of claims 1 or 2, wherein at least two of the candidate biochemical entities have a similar pharmacological property or functional effect.

4. The method of any one of claims 1 to 3, wherein the intrinsically disordered protein is alpha-synuclein.

5. The method of any one of claims 1 to 4, wherein the signature of one or more of the candidate biochemical entities correlates to a known pharmacological property or a known functional effect.

6. The method of any one of claims 1 to 5, wherein the intrinsically disordered protein is labeled with a nonlinear-active unnatural amino acid.

7. The method of claim 6, wherein the nonlinear-active unnatural amino acid is Aladan.

## Patentansprüche

1. Verfahren für das Screening biochemischer Einheiten, das Verfahren umfassend:
(a) Bereitstellen einer Bibliothek, umfassend 6 oder mehr Kandidaten der biochemischen Einheiten;
(b) Messen eines ersten konformativen Zustands eines intrinsisch ungeordneten Proteins, dadurch Erzeugen einer Vergleichslinie;
(c) in Kontakt bringen jedes der Kandidaten der biochemischen Einheiten mit demselben intrinsisch ungeordneten Protein;
(d) Messen eines zweiten konformativen Zustands eines intrinsisch ungeordneten Proteins;
(e) Bestimmen einer Signatur für mindestens einen ersten und einen zweiten Kandidaten der biochemischen Einheiten unter Verwendung des zweiten konformativen Zustands
(f) Vergleichen der Signatur des ersten Kandidaten der biochemischen Einheiten mit der Signatur des zweiten Kandidaten der biochemischen Einheiten; und (g)Auswählen, basierend auf dem Vergleich in (f), einer oder mehrerer biochemischer Einheiten aus den Kandidaten der biochemischen Einheiten,
wobei die Kandidaten der biochemischen Einheiten kleine Moleküle sind, die einen gemeinsamen Gerüstkern teilen, und wobei die Messschritte Messungen einer Frequenzverdopplung (SHG) umfassen.

2. Das Verfahren nach Anspruch 1, wobei jeder der Kandidaten der biochemischen Einheiten eine andere Veränderung des konformativen Zustands des intrinsisch ungeordneten Proteins hervorbringt.

3. Das Verfahren nach einem der Ansprüche 1 oder 2, wobei mindestens zwei der Kandidaten der biochemischen Einheiten eine ähnliche pharmakologische Eigenschaft oder Funktionswirkung aufweisen.

4. Das Verfahren nach einem der Ansprüche 1 bis 3, wobei das intrinsisch ungeordnete Protein ein alpha-Synuclein ist.

5. Das Verfahren nach einem der Ansprüche 1 bis 4, wobei die Signatur eines oder mehrerer der Kandidaten der biochemischen Einheiten mit einer bekannten pharmakologischen Eigenschaft oder einer bekannten Funktionswirkung korreliert.

6. Das Verfahren nach einem der Ansprüche 1 bis 5, wobei das intrinsisch ungeordnete Protein mit einer nichtlinearen-aktiven unnatürlichen Aminosäure markiert ist.

7. Das Verfahren nach Anspruch 6, wobei die nichtlineare-aktive unnatürliche Aminosäure Aladan ist.

## Revendications

1. Procédé de dépistage d'entités biochimiques, le procédé comprenant :
(a) la fourniture d'une bibliothèque comprenant 6 ou plus entités biochimiques candidates ;
(b) la mesure d'un premier état conformationnel d'une protéine intrinsèquement désordonnée, ce qui permet de générer un niveau de référence ;
(c) la mise en contact de chacune des entités biochimiques candidates avec la même protéine intrinsèquement désordonnée ;
(d) la mesure d'un second état conformationnel de la protéine intrinsèquement désordonnée ;
(e) la détermination d'une signature pour au moins une première et une seconde entité biochimique candidate à l'aide du second état conformationnel ;
(f) la comparaison de la signature de la première entité biochimique candidate à la signature de la seconde entité biochimique candidate ; et
(g) la sélection, sur la base de la comparaison dans (f), d'une ou plusieurs entités biochimiques à partir des entités biochimiques candidates,
dans lequel les entités biochimiques candidat sont de petites molécules qui partagent un tuteur commun et dans lequel les étapes de mesure comprennent les mesures de génération de la seconde harmonique (SHG).

2. Procédé selon la revendication 1, dans lequel, chacune des entités biochimiques candidates produit un changement d'état conformationnel différent de la protéine intrinsèquement désordonnée.

3. Procédé selon l'une quelconque des revendications 1 ou 2, dans lequel au moins deux des entités biochimiques candidates présentent une propriété pharmacologique ou un effet fonctionnel similaire.

4. Procédé selon l'une quelconque des revendications 1 à 3, dans lequel la protéine intrinsèquement désordonnée est alpha-synucléine.

5. Procédé selon l'une quelconque des revendications 1 à 4, dans lequel la signature d'une ou plusieurs parmi les entités biochimiques candidates correspond à une propriété pharmacologique connue ou à un effet fonctionnel connu.

6. Procédé selon l'une quelconque des revendications 1 à 5, dans lequel la protéine intrinsèquement désordonnée est étiquetée avec un acide aminé non naturel non linéaire actif.

7. Procédé selon la revendication 6, dans lequel l'acide aminé non naturel non linéaire actif est aladan.
